# EUROPEAN PATENT SPECIFICATION

(11) **EP 2 521 734 B1**
(45) Date of publication and mention of the grant of the patent: **14.09.2016**
(21) Application number: 10724120.0
(22) Date of filing: 08.06.2010
(51) Int. Cl.: C07K 14/415, C12N 15/82, A01H 5/00

(54) **METHODS AND COMPOSITIONS FOR ALTERING TEMPERATURE SENSING IN EUKARYOTIC ORGANISMS**
VERFAHREN UND ZUSAMMENSETZUNGEN ZUR VERÄNDERUNG DER TEMPERATURERFASSUNG IN EUKARYOTISCHER ORGANISMEN
PROCÉDÉS ET COMPOSITIONS POUR MODIFIER LA DÉTECTION DE TEMPÉRATURE CHEZ DES ORGANISMES EUCARYOTES

(30) Priority: 07.01.2010 GB 201000184
(43) Date of publication of application: 14.11.2012
(73) Proprietor: Plant Bioscience Limited, Norwich, Norfolk NR4 7UH (GB)
(72) Inventor: WIGGE, Philip, Norwich Norfolk NR4 7UH (GB); KUMAR, Vinod, Norwich Norfolk NR4 7UH (GB)
(74) Representative: Williams, Andrea
(86) International application number: PCT/GB2010/050956
(87) International publication number: WO 2011/083290

(56) References cited:
- EP-A2- 1 094 112
- KUMAR S V ET AL: "H2A.Z-Containing Nucleosomes Mediate the Thermosensory Response in Arabidopsis" CELL 20100108 CELL PRESS USA LNKD- DOI:10.1016/J.CELL.2009.11.006, vol. 140, no. 1, 8 January 2010 (2010-01-08), pages 136-147, XP002592866 ISSN: 0092-8674
- ISHIBASHI TOYOTAKA ET AL: "Acetylation of vertebrate H2A.Z and its effect on the structure of the nucleosome." BIOCHEMISTRY 9 JUN 2009 LNKD- PUBMED:19385636, vol. 48, no. 22, 9 June 2009 (2009-06-09), pages 5007-5017, XP002592867 ISSN: 1520-4995
- MILLAR CATHERINE B ET AL: "Acetylation of H2AZ Lys 14 is associated with genome-wide gene activity in yeast" GENES & DEVELOPMENT, vol. 20, no. 6, March 2006 (2006-03), pages 711-722, XP002592868 ISSN: 0890-9369
- MARCH-DIAZ R ET AL: "Histone H2A.Z and homologues of components of the SWR1 complex are required to control immunity in Arabidopsis" PLANT JOURNAL FEBRUARY 2008 BLACKWELL PUBLISHING LTD GB, vol. 53, no. 3, February 2008 (2008-02), pages 475-487, XP002592869 DOI: DOI:10.1111/J.1365-313X.2007.03361.X
- MARCH-DÍAZ ROSANA ET AL: "The beauty of being a variant: H2A.Z and the SWR1 complex in plants." MOLECULAR PLANT JUL 2009 LNKD- PUBMED:19825639, vol. 2, no. 4, July 2009 (2009-07), pages 565-577, XP002592870 ISSN: 1674-2052

### SEQUENCE LISTING

<110> Plant Bioscience Limited
<120> Methods and compositions for altering temperature sensing in eukaryotic organisms
<130> PC925279WO
<150> GB1000184.0
   <151> 2010-01-07
<160> 16
<170> PatentIn version 3.3
<210> 1
   <211> 22
   <212> DNA
   <213> Artificial
<220>
   <223> Oligo primer W1160
<400> 1
   caccgaaatg tttttctcta cg 22
<210> 2
   <211> 20
   <212> DNA
   <213> Artificial
<220>
   <223> Oligo primer W1161
<400> 2
   ctccttggtg gttttgttga 20
<210> 3
   <211> 45
   <212> DNA
   <213> Artificial
<220>
   <223> Oligo primer W1443
<400> 3
   tcaagagaca tggcaggcag aggtggaaga ggactcgtag ctgcg 45
<210> 4
   <211> 45
   <212> DNA
   <213> Artificial
<220>
   <223> Oligo primer W1444
<400> 4
   cgcagctacg agtcctcttc cacctctgcc tgccatgtct cttga 45
<210> 5
   <211> 33
   <212> DNA
   <213> Artificial
<220>
   <223> Oligo primer W1445
<400> 5
   ggactcgtag ctgcgaggac gatggctgct aac 33
<210> 6
   <211> 33
   <212> DNA
   <213> Artificial
<220>
   <223> Oligo primer W1446
<400> 6
   gttagcagcc atcgtcctcg cagctacgag tcc 33
<210> 7
   <211> 40
   <212> DNA
   <213> Artificial
<220>
   <223> Oligo primer W1447
<400> 7
   gacgatggct gctaacaggg acagagacaa ggacaagaag 40
<210> 8
   <211> 40
   <212> DNA
   <213> Artificial
<220>
   <223> Oligo primer W1448
<400> 8
   cttcttgtcc ttgtctctgt ccctgttagc agccatcgtc 40
<210> 9
   <211> 139
   <212> PRT
   <213> Oryza sativa
<400> 9
   Met Ala Gly Lys Gly Gly Lys Gly Leu Leu Ala Ala Lys Thr Thr Ala
   1               5                   10                  15
   Ala Lys Ala Ala Ala Asp Lys Asp Lys Asp Arg Lys Lys Ala Pro Val
               20                  25                  30
   Ser Arg Ser Ser Arg Ala Gly Ile Gln Phe Pro Val Gly Arg Ile His
           35                  40                  45
   Arg Gln Leu Lys Gly Arg Val Ser Ala Asn Gly Arg Val Gly Ala Thr
       50                  55                  60
   Ala Ala Val Tyr Thr Ala Ala Ile Leu Glu Tyr Leu Thr Ala Glu Val
   65                  70                  75                  80
   Leu Glu Leu Ala Gly Asn Ala Ser Lys Asp Leu Lys Val Lys Arg Ile
                   85                  90                  95
   Thr Pro Arg His Leu Gln Leu Ala Ile Arg Gly Asp Glu Glu Leu Asp
               100                 105                 110
   Thr Leu Ile Lys Gly Thr Ile Ala Gly Gly Gly Val Ile Pro His Ile
           115                 120                 125
   His Lys Ser Leu Ile Asn Lys Thr Ala Lys Glu
       130                 135
<210> 10
   <211> 138
   <212> PRT
   <213> Oryza sativa
<400> 10
   Met Ala Gly Lys Gly Gly Lys Gly Leu Leu Ala Ala Lys Thr Thr Ala
   1               5                   10                  15
   Ala Lys Ser Ala Asp Lys Asp Lys Asp Lys Lys Lys Ala Pro Val Ser
               20                  25                  30
   Arg Ser Ser Arg Ala Gly Leu Gln Phe Pro Val Gly Arg Ile His Arg
           35                  40                  45
   Gln Leu Lys Ser Arg Ala Ser Ala His Gly Arg Val Gly Ala Thr Ala
       50                  55                  60
   Ala Val Tyr Ser Ala Ala Ile Leu Glu Tyr Leu Thr Ala Glu Val Leu
   65                  70                  75                  80
   Glu Leu Ala Gly Asn Ala Ser Lys Asp Leu Lys Val Lys Arg Ile Thr
                   85                  90                  95
   Pro Arg His Leu Gln Leu Ala Ile Arg Gly Asp Glu Glu Leu Asp Thr
               100                 105                 110
   Leu Ile Lys Gly Thr Ile Ala Gly Gly Gly Val Ile Pro His Ile His
           115                 120                 125
   Lys Ser Leu Ile Asn Lys Thr Ser Lys Glu
       130                 135
<210> 11
   <211> 137
   <212> PRT
   <213> Oryza sativa
<400> 11
   Met Ala Gly Lys Gly Gly Lys Gly Leu Leu Ala Ala Lys Thr Thr Ala
   1               5                   10                  15
   Ala Lys Ser Ala Glu Lys Asp Lys Gly Lys Lys Ala Pro Val Ser Arg
               20                  25                  30
   Ser Ser Arg Ala Gly Leu Gln Phe Pro Val Gly Arg Ile His Arg Gln
           35                  40                  45
   Leu Lys Gln Arg Thr Gln Ala Asn Gly Arg Val Gly Ala Thr Ala Ala
       50                  55                  60
   Val Tyr Ser Ala Ala Ile Leu Glu Tyr Leu Thr Ala Glu Val Leu Glu
   65                  70                  75                  80
   Leu Ala Gly Asn Ala Ser Lys Asp Leu Lys Val Lys Arg Ile Thr Pro
                   85                  90                  95
   Arg His Leu Gln Leu Ala Ile Arg Gly Asp Glu Glu Leu Asp Thr Leu
               100                 105                 110
   Ile Lys Gly Thr Ile Ala Gly Gly Gly Val Ile Pro His Ile His Lys
           115                 120                 125
   Ser Leu Ile Asn Lys Ser Ser Lys Glu
       130                 135
<210> 12
   <211> 134
   <212> PRT
   <213> Arabidopsis thaliana
<400> 12
   Met Ser Gly Lys Gly Ala Lys Gly Leu Ile Met Gly Lys Pro Ser Gly
   1               5                   10                  15
   Ser Asp Lys Asp Lys Asp Lys Lys Lys Pro Ile Thr Arg Ser Ser Arg
               20                  25                  30
   Ala Gly Leu Gln Phe Pro Val Gly Arg Val His Arg Leu Leu Lys Thr
           35                  40                  45
   Arg Ser Thr Ala His Gly Arg Val Gly Ala Thr Ala Ala Val Tyr Thr
       50                  55                  60
   Ala Ala Ile Leu Glu Tyr Leu Thr Ala Glu Val Leu Glu Leu Ala Gly
   65                  70                  75                  80
   Asn Ala Ser Lys Asp Leu Lys Val Lys Arg Ile Ser Pro Arg His Leu
                   85                  90                  95
   Gln Leu Ala Ile Arg Gly Asp Glu Glu Leu Asp Thr Leu Ile Lys Gly
   100 105 110
   Thr Ile Ala Gly Gly Gly Val Ile Pro His Ile His Lys Ser Leu Ile
   115 120 125
   Asn Lys Ser Ala Lys Glu
   130
<210> 13
   <211> 136
   <212> PRT
   <213> Arabidopsis thaliana
<400> 13
   Met Ala Gly Lys Gly Gly Lys Gly Leu Leu Ala Ala Lys Thr Thr Ala
   1 5 10 15
   Ala Ala Ala Asn Lys Asp Ser Val Lys Lys Lys Ser Ile Ser Arg Ser
   20 25 30
   Ser Arg Ala Gly Ile Gln Phe Pro Val Gly Arg Ile His Arg Gln Leu
   35 40 45
   Lys Gln Arg Val Ser Ala His Gly Arg Val Gly Ala Thr Ala Ala Val
   50 55 60
   Tyr Thr Ala Ser Ile Leu Glu Tyr Leu Thr Ala Glu Val Leu Glu Leu
   65 70 75 80
   Ala Gly Asn Ala Ser Lys Asp Leu Lys Val Lys Arg Ile Thr Pro Arg
   85 90 95
   His Leu Gln Leu Ala Ile Arg Gly Asp Glu Glu Leu Asp Thr Leu Ile
   100 105 110
   Lys Gly Thr Ile Ala Gly Gly Gly Val Ile Pro His Ile His Lys Ser
   115 120 125
   Leu Val Asn Lys Val Thr Lys Asp
   130 135
<210> 14
   <211> 136
   <212> PRT
   <213> Arabidopsis thaliana
<400> 14
   Met Ala Gly Lys Gly Gly Lys Gly Leu Val Ala Ala Lys Thr Met Ala
   1               5                   10                  15
   Ala Asn Lys Asp Lys Asp Lys Asp Lys Lys Lys Pro Ile Ser Arg Ser
               20                  25                  30
   Ala Arg Ala Gly Ile Gln Phe Pro Val Gly Arg Ile His Arg Gln Leu
           35                  40                  45
   Lys Thr Arg Val Ser Ala His Gly Arg Val Gly Ala Thr Ala Ala Val
       50                  55                  60
   Tyr Thr Ala Ser Ile Leu Glu Tyr Leu Thr Ala Glu Val Leu Glu Leu
   65                  70                  75                  80
   Ala Gly Asn Ala Ser Lys Asp Leu Lys Val Lys Arg Ile Thr Pro Arg
                   85                  90                  95
   His Leu Gln Leu Ala Ile Arg Gly Asp Glu Glu Leu Asp Thr Leu Ile
               100                 105                 110
   Lys Gly Thr Ile Ala Gly Gly Gly Val Ile Pro His Ile His Lys Ser
           115                 120                 125
   Leu Ile Asn Lys Thr Thr Lys Glu
       130                 135
<210> 15
   <211> 132
   <212> PRT
   <213> Zea mays
<400> 15
   Met Ala Gly Lys Gly Gly Gly Lys Ser Leu Val Ala Ala Lys Ile Ala
   1               5                   10                  15
   Ala Gly Met Asp Lys Asp Lys Lys Ser Pro Val Asn Arg Ser Ala Arg
               20                  25                  30
   Ala Gly Leu His Phe Pro Val Gly Arg Ile His Arg His Leu Lys Asp
           35                  40                  45
   Ser Ala Gln Ala Gly Glu Arg Val Gly Ala Thr Ala Ala Val Tyr Ala
       50                  55                  60
   Ala Ala Val Leu Glu Tyr Leu Thr Ala Glu Ile Met Glu Leu Ala Gly
   65                  70                  75                  80
   Asn Ala Cys Arg Asp Leu Lys Val Lys Arg Ile Thr Pro Arg His Leu
                   85                  90                  95
   Gln Leu Ala Val Arg Gly Asp Glu Glu Leu Asp Thr Leu Ile Lys Ala
               100                 105                 110
   Thr Ile Ala Gly Gly Gly Val Ile Pro His Ile His Arg Glu Leu Ile
           115                 120                 125
   Asp Lys Ala Ala
       130
<210> 16
   <211> 138
   <212> PRT
   <213> Zea mays
<400> 16
   Met Ala Gly Lys Gly Gly Lys Gly Leu Leu Ala Ala Lys Thr Thr Ala
   1               5                   10                  15
   Ala Lys Ser Thr Asp Lys Asp Lys Asp Arg Lys Lys Ala Pro Val Ser
               20                  25                  30
   Arg Ser Ser Arg Ala Gly Leu Gln Phe Pro Val Gly Arg Ile His Arg
           35                  40                  45
   Gln Leu Lys Ser Arg Ala Ser Ala His Gly Arg Val Gly Ala Thr Ala
       50                  55                  60
   Ala Val Tyr Ser Ala Ala Ile Leu Glu Tyr Leu Thr Ala Glu Val Leu
   65                  70                  75                  80
   Glu Leu Ala Gly Asn Ala Ser Lys Asp Leu Lys Val Lys Arg Ile Thr
                   85                  90                  95
   Pro Arg His Leu Gln Leu Ala Ile Arg Gly Asp Glu Glu Leu Asp Thr
               100                 105                 110
   Leu Ile Lys Gly Thr Ile Ala Gly Gly Gly Val Ile Pro His Ile His
           115                 120                 125
   Lys Ser Leu Ile Asn Lys Thr Ala Lys Glu
       130                 135

## Description

### FIELD OF THE INVENTION

H2A.Z-containing nucleosomes mediate the thermosensory response in eukaryotes and modifications to H2A.Z alter this response.

### BACKGROUND OF THE INVENTION

Sessile organisms, such as plants, continually sense environmental conditions to adapt their growth and development. Temperature varies both diurnally, which is important for entraining the clock (Michael et al., 2008; Salome and McClung, 2005), as well as seasonally, providing information for the timing of reproduction (Heggie and Halliday, 2005; Samach and Wigge, 2005; Sung and Amasino, 2005). Extremes of temperature represent a significant stress for plants, and are a major factor limiting global plant distribution (Mittler, 2006). The sensitivity of plants to small changes in temperature is highlighted by significant changes in flowering time (Fitter and Fitter, 2002) and distributions of wild-plants that have occurred in the last 100 years due to climate change (Kelly and Goulden, 2008; Lenoir et al., 2008; Willis et al., 2008). Projected increases in mean global temperature, as well as extremes of temperature in the next 100 years, are significantly larger than what has occurred so far, suggesting significant future disruption to wild plants and crops.

Plants must constantly respond to changes in the environment whilst maintaining developmental and growth processes if they are to survive into the next generation. A complex network of signals from temperature and light must correctly converge to achieve successful development, through vegetative to reproductive growth. Temperature can be thought of as an environmental factor that provides both 'inductive' and 'maintenance' signals in development. It can stimulate developmental processes such as seed dormancy release, germination, vernalization and flowering. Thus, being able to control how a plant perceives temperature is desirable. The responses of organisms to temperature, particularly in crop plants, are often detrimental, and the targeted alteration of these responses may improve the yield, quality and predictability of crops. For example grain-fill in wheat and rice is particularly sensitive to high temperatures. Preventing a heat shock response in the developing grain would protect grain composition and quantity from being perturbed by a high temperature stress response. High temperature stress responses have been selected for in evolution and breeding in order to maximise embryo survival, but these responses are detrimental to grain quality. This is already a major cause of reduced wheat and rice yields during hot summers, and these problems will be exacerbated by future climate change.

Additionally many crops flower or bolt prematurely when exposed to high or low temperatures (for example sugar beet, lettuce, onions, cabbage and broccoli), which is a major cause of crop loss. Specifically controlling the flowering responses to temperature in such crops is desirable. Furthermore, reducing temperature sensitivity may enable a more uniform development within a crop, which is important, since it enables farmers to schedule harvests reliably and optimises crop yield and quality. Additionally, many harmful eukaryotic micro-organisms use temperature perception as a signal to induce pathogenesis, for example *Histoplasma capsulatum* and *Candida albicans* (Nguyen and Sil, 2008). The ability to selectively perturb temperature perception in these organisms would provide means to prevent pathogenesis.

Temperature sensing in eukaryotes is a complex process that is not yet fully understood. In plants, factors that are involved in temperature sensing include calcium signalling and low temperature is known to change membrane fluidity.

Genetic information within the eukaryotic cell nucleus is organized in a highly conserved structural polymer, the chromatin, which supports and controls crucial functions of the genome. The basic unit of chromatin is the nucleosome, which contains DNA wrapped around a histone octamer. Core histones H2A, H2B, H3, and H4 have a highly structured core domain involved in intranucleosomal histone-histone interactions and unstructured N- and C-terminal tails that extend outside of the nucleosomal core, and they interact with other nucleosomes and with nonhistone proteins. These tails are subject to a number of post-translational modifications, such as acetylation, methylation, phosphorylation, and ubiquitination. The organization of DNA into chromatin is inhibitory to all the processes that need DNA as a template, such as replication, transcription, recombination, and repair. There are a number of mechanisms by which chromatin structure and composition can be manipulated to facilitate these events. These include post-translational modifications of histones. These modifications can alter DNA-histone interactions within and between nucleosomes and, thus, affect higher-order chromatin structures. Another mechanism of modulating chromatin structure is carried out by multisubunit ATP-dependent chromatin remodelling complexes.

In addition to genes encoding the core histone H2A, the eukaryotic genome contains genes referred to as H2A variants, which have a different protein sequences from core H2A. Histone variants play critical roles in chromatin structure and function and are implicated in a variety of cellular activities, such as regulation of transcription, DNA repair, chromosome X inactivation, and heterochromatin formation. H2A.Z variants are highly conserved from *Saccharomyces cerevisiae* to human. H2A.Z variants have also been identified in plants.

In summary, being able to control temperature regulated responses in plants is of major importance in controlling yield, in particular in view of climate change.

Moreover, being able to regulate temperature responses in other eukaryotic organisms, such as yeast is also desirable.

We have identified methods and compositions which are aimed at meeting this need.

### SUMMARY OF THE INVENTION

We have shown that temperature responses in eukaryotic cells are mediated by the presence of H2A.Z in the nucleosome, and we disclose herein ways in which temperature perception may be specifically altered to optimise responses of plants, yeast, fungi or other eukaryotic organisms. We have found that H2A.Z-containing nucleosomes represent the major node of regulation of responses to temperature and to temperature changes in plants.

We have demonstrated that the gene transcription of temperature responsive genes is coordinated by H2A.Z-containing nucleosomes in plants. We have shown that the presence of H2A.Z in the nucleosome decreases with an increase in temperature, and that this effect is independent of transcription. Without H2A.Z being present in the nucleosome, plants display a constitutive warm temperature response, developmentally and transcriptionally. Thus, the disclosure relates to methods for altering temperature sensing in a eukaryotic organism and to producing a eukaryotic organism with altered temperature sensing. The disclosure also relates to methods for altering thermosensory responses in a eukaryotic organism and to producing a eukaryotic organism with altered thermosensory responses.

In a first aspect, the invention relates to a method for altering temperature sensing in a plant or a part thereof comprising modifying the presence or state of H2A.Z in the nucleosome by decreasing or increasing the level of H2A.Z in said plant or a part thereof or altering post-translational modification of H2A.Z, wherein said post-translational modification is acetylation.

In another aspect, the invention relates to a method for altering thermosensory responses in a plant said method comprising:
a) producing a mutant H2A.Z protein wherein said mutant H2A.Z protein has no acetylation sites, a reduced number of acetylation sites or increased acetylation sites, wherein said acetylation site comprises a codon encoding a lysine (K) residue; or
b) altering the expression of a nucleic acid sequence encoding a histone deacetylase (HDAC) enzyme or altering the activity of HDAC
or combinations thereof.

In a third aspect, the invention relates to a method for inhibiting the response of a plant to an increase in ambient temperature comprising increasing the presence of H2A.Z in the nucleosome or preventing release of H2A.Z from the nucleosome, altering the state of H2A.Z, such as post-translational modification of H2A.Z, or inhibiting disassembly of the H2A.Z from the nucleosome.

We also describe a method for eliciting a warm temperature response in a eukaryotic organism comprising inhibiting or decreasing the presence H2A.Z in the nucleosome.

We also describe a method for eliciting a cold temperature response comprising inhibiting or decreasing the eviction of H2A.Z from the nucleosome.

We also describe a method for producing a eukaryotic organism that shows a constitutive warm temperature response comprising inhibiting or decreasing the presence H2A.Z in the nucleosome.

We also describe a method for producing a eukaryotic organism that shows a constitutive cold temperature response comprising inhibiting or decreasing the eviction of H2A.Z from the nucleosome.

Other objects and advantages of this invention will be appreciated from a review of the complete disclosure provided herein and the appended claims.

### BRIEF DESCRIPTION OF THE DRAWINGS

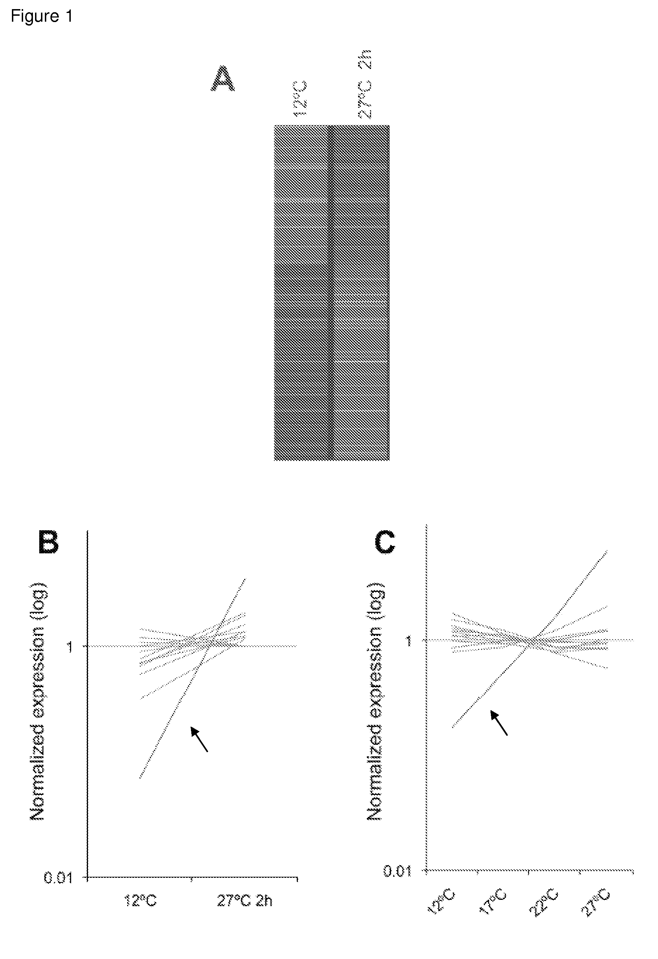

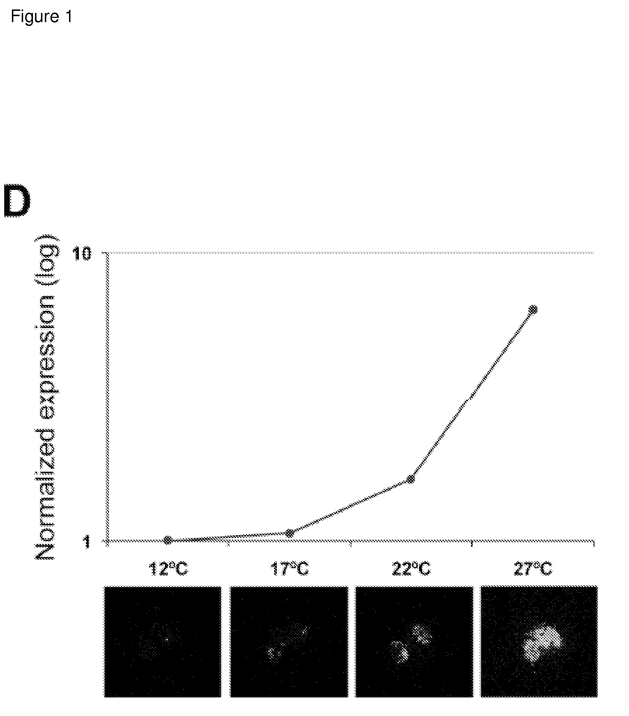

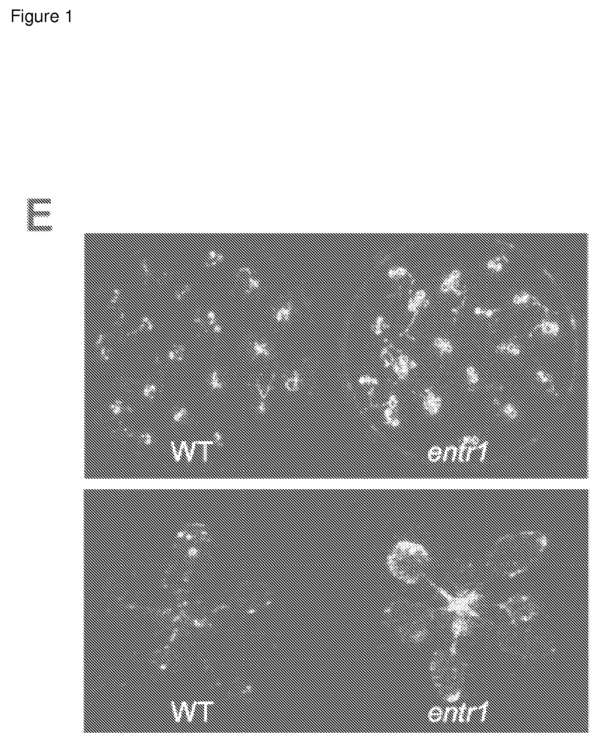

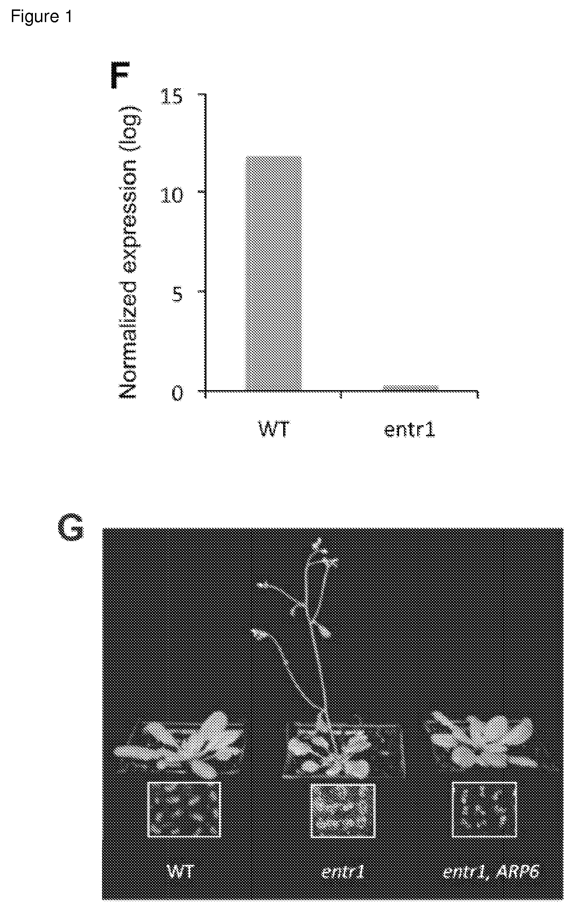

### Figure 1. HSP70 expression is an output of the ambient temperature sensing pathway.

(A) Transcript profiling experiments show a robust transcriptional response to changes in ambient temperature.
(B) *HSP70* (black line, marked with an arrow) is induced strongly with increasing ambient temperature. In contrast, the other members of the *HSP70* family genes (gray) require a heat stress to be up-regulated.
(C) *HSP70* (black line, marked with an arrow) has a uniform linear expression pattern at various constant growth temperatures, in contrast to the rest of the *HSP70* family (gray). *HSP70* is therefore an excellent output of the thermosensory pathway over a wide temperature range.
(D) *HSP70::LUC* in Arabidopsis mimics the endogenous *HSP70* expression pattern in response to temperature. *LUC* transcript analysis and live luciferase imaging of plants shifted to 17°C, 22°C and 27°C for two hours from 12°C show a temperature dependent LUC expression and luciferase activity. *LUC* transcript levels were normalised to *UBQ10.*
(E) Identification of *entr1* through a forward genetic screen as a mutant with enhanced temperature response at the seedling stage (upper panel), this phenotype is present throughout the life cycle. Lower panel shows the *HSP70::LUC* expression in adult wild-type and *entr1* plants.
(F) Transcript profiling analysis showing *ARP6* gene expression in WT and *entr1. ARP6* transcripts were absent in *entr1.* The bar represents normalised expression levels from the microarray experiment.
(G) Complementation of *entr1:* a binary construct containing the *ARP6* genomic fragment including the native promoter and coding regions (*P_{ARP6}::ARP6*) completely rescued the *HSP70::LUC* as well as the developmental phenotypes of *entr1.*

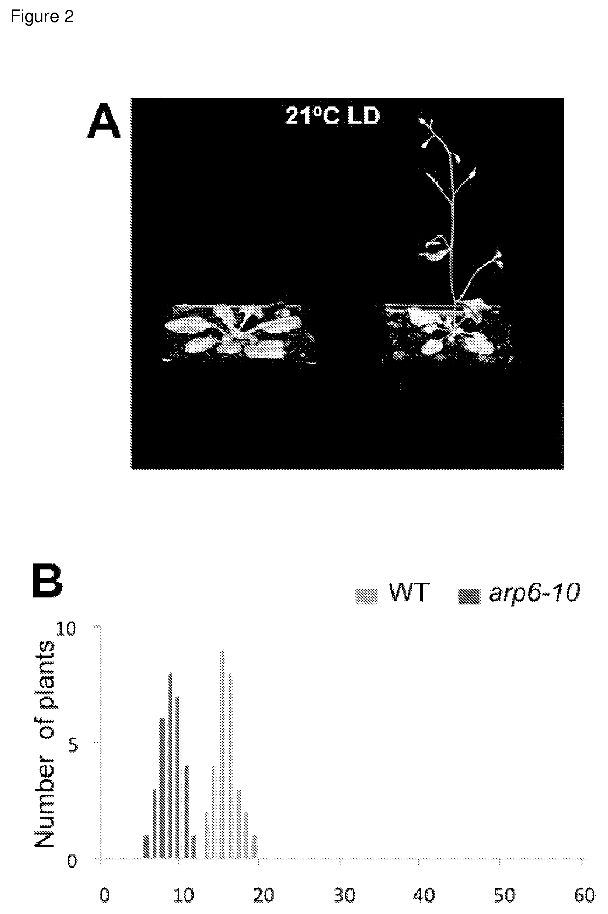

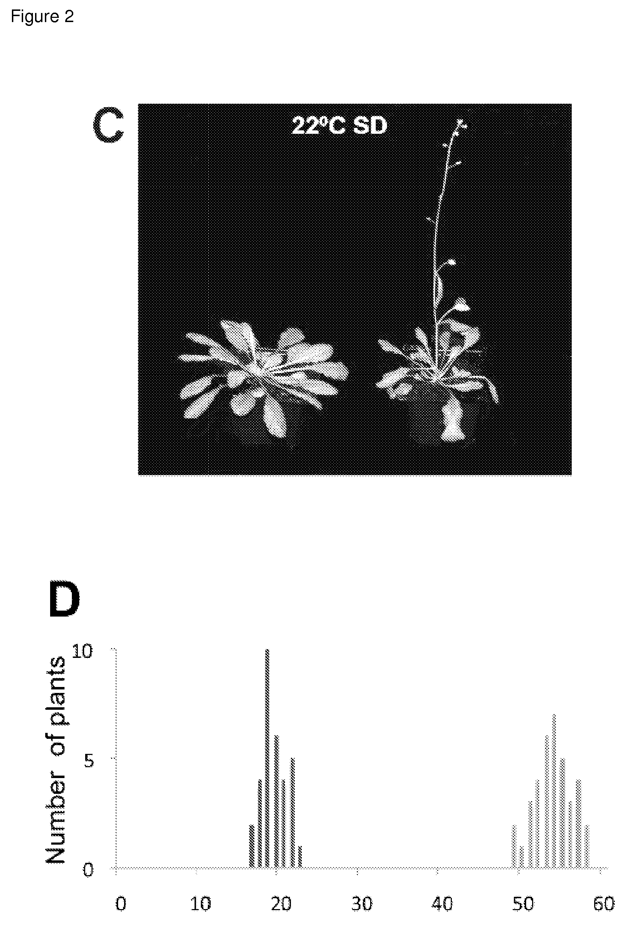

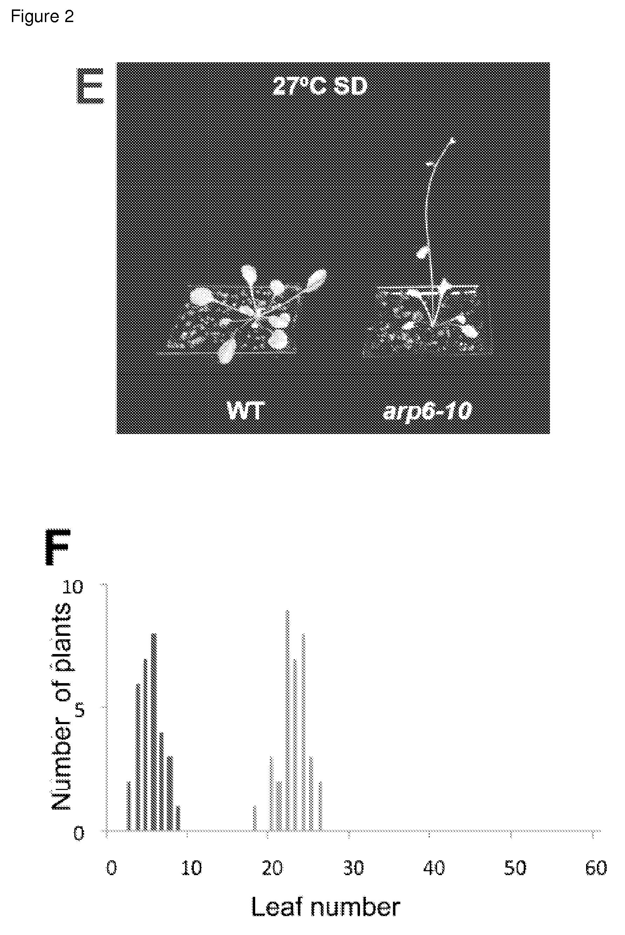

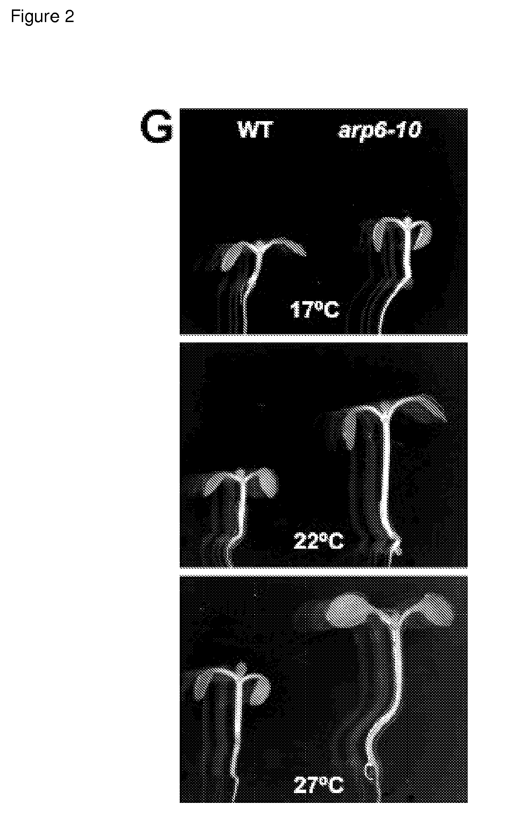

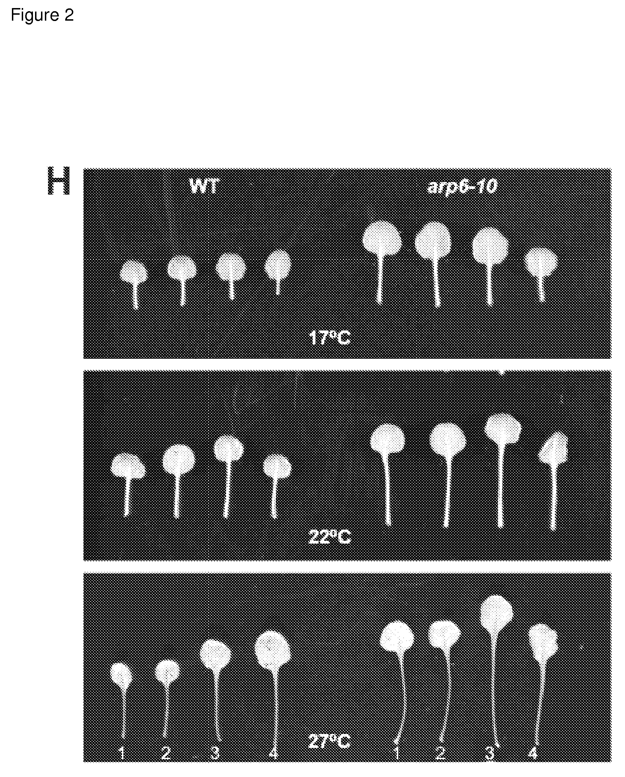

### Figure 2. arp6-10 displays developmental and architectural phenotypes of warm grown plants.

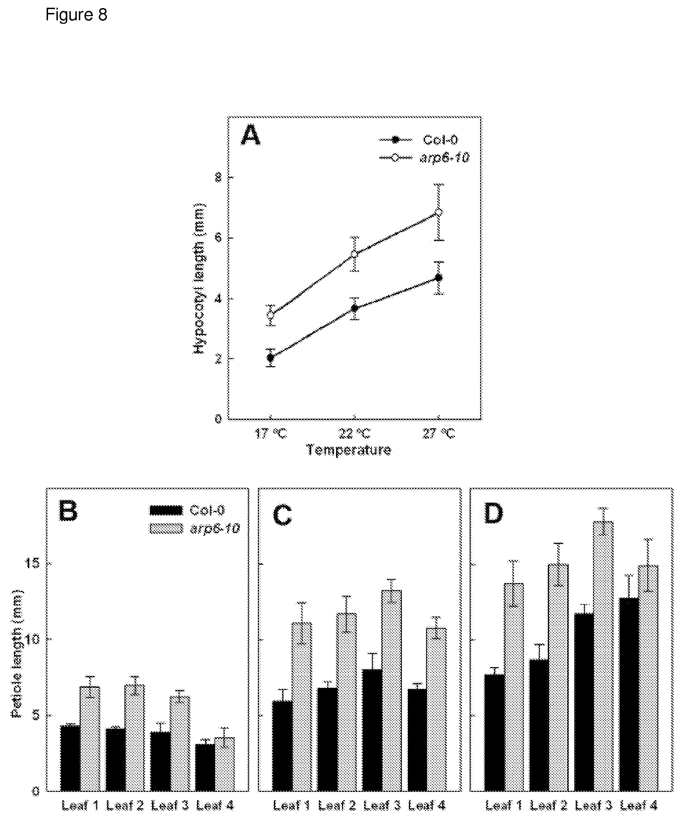

(A-D) *arp6*-*10* flowers significantly earlier than wild-type under long-day (A, B) and short day conditions (C, D) at 22 °C. When grown in short days at 27 °C (E, F), *arp6*-*10* shows a very strong thermal induction of flowering. In comparison, wild-type flowering time is similar to *arp6*-*10* at 22 °C. (x-axis shows the number of rosette leaves at the time of flowering, y-axis shows the corresponding number of plants). (G, H) In addition to the flowering time phenotypes, *arp6*-*10* displays all the architectural responses of wild-type plants grown at high temperature. *arp6*-*10* seedlings at 17°C, show hypocotyl (G) and petiole elongation (H) that is equivalent to the wild-type phenotype at higher temperatures. (Petiole elongation was analysed on the first four true leaves of 20-day-old wild-type and *arp6*-*10* seedlings). See also Figure 8.

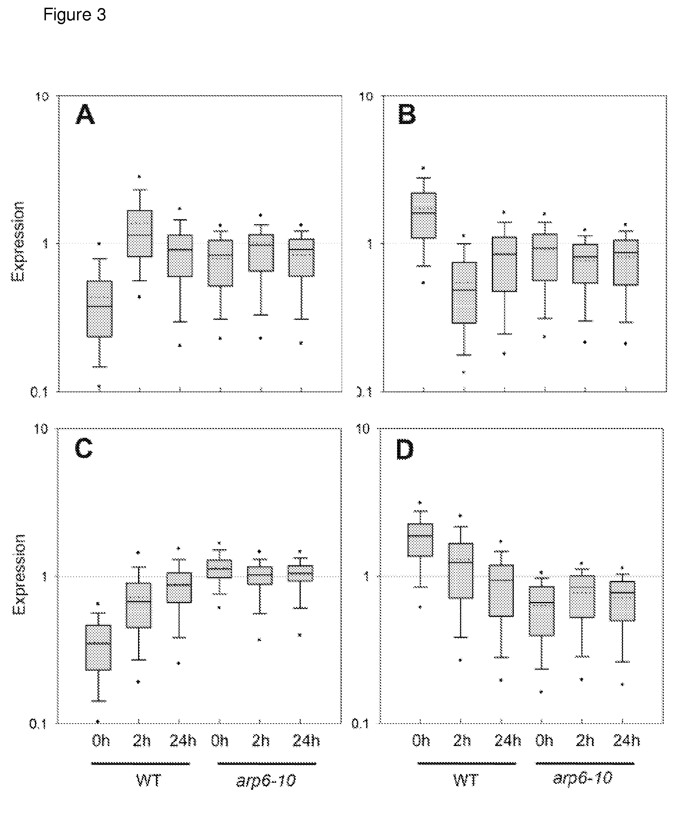

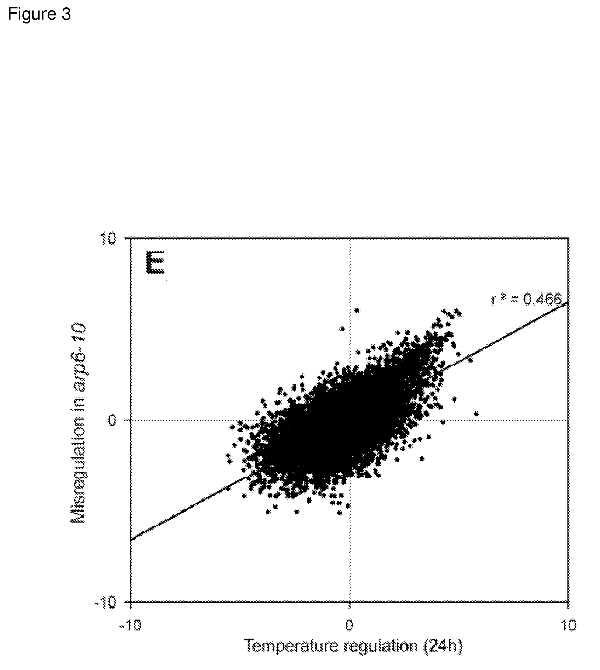

### Figure 3. The temperature transcriptome is globally mis-regulated in arp6-10.

Genes that are 2 fold up- (A) or down-regulated (B) in wild-type seedling within 2 hours of shifting to 27 °C are constitutively repressed or activated in the *arp6*-*10* background. Similarly genes that are 2 fold up- (C) or down-regulated (D) in *arp6*-*10* compared to the wild-type at 12 °C (0 h) were down- or up-regulated in the wild-type upon shift to 27 °C. Median and mean values are represented respectively by the solid and dotted horizontal lines. 5th and 95th percentiles represent outlier values (dots).
(E) Comparison of *arp6*-*10* induced genome-wide transcriptional changes with responses to increasing temperature from 12 °C to 27 °C in wild-type. Whole genome scatter plot analysis of expression log ratios. x-axis: comparison of wild-type after 24h at 27 °C with seedlings kept at 12 °C. y-axis: comparison of *arp6*-*10* grown at 12 °C with wild-type at 12 °C. The *arp6*-*10* mutation accounts for nearly half (R² = 0.466) of the changes in the transcriptome in response to an increase in ambient temperature.

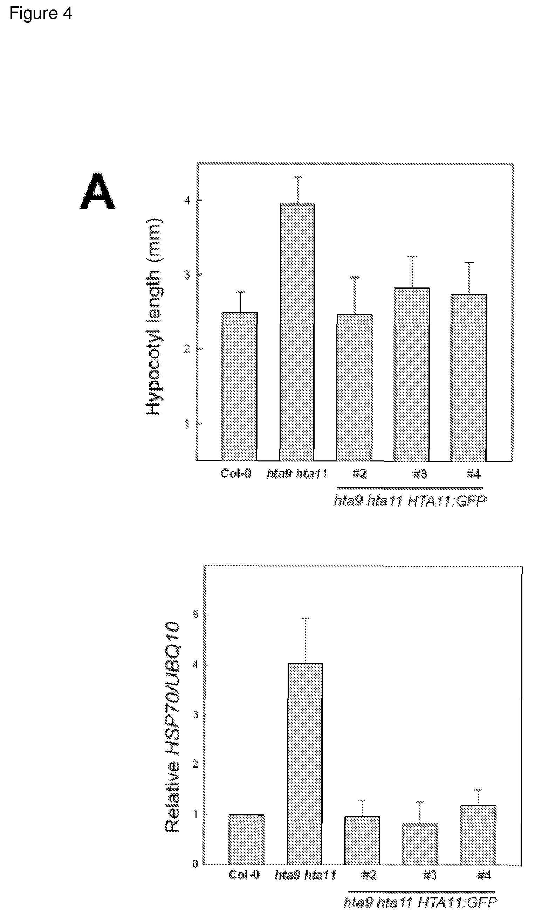

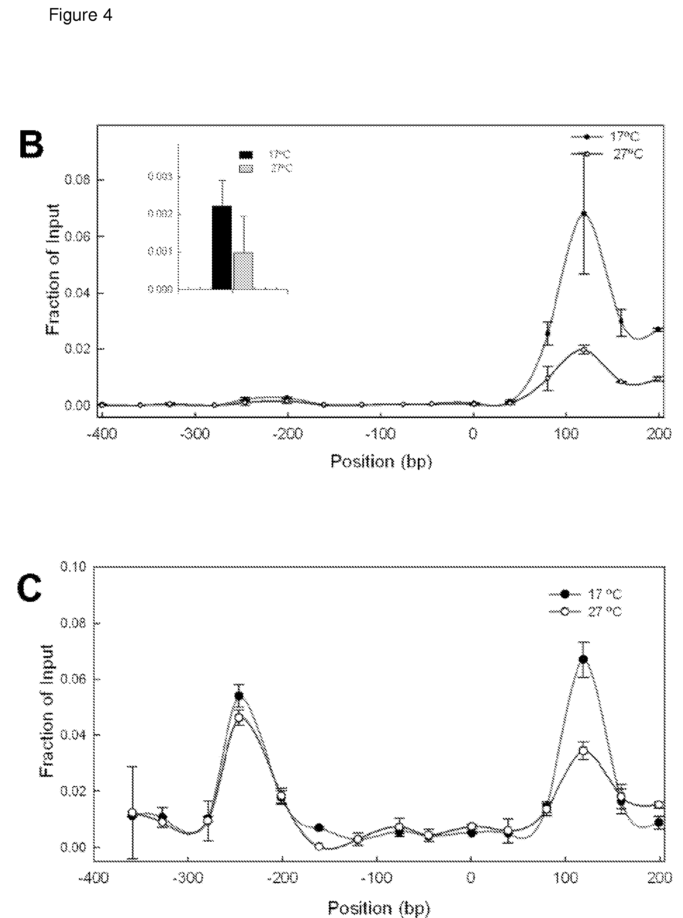

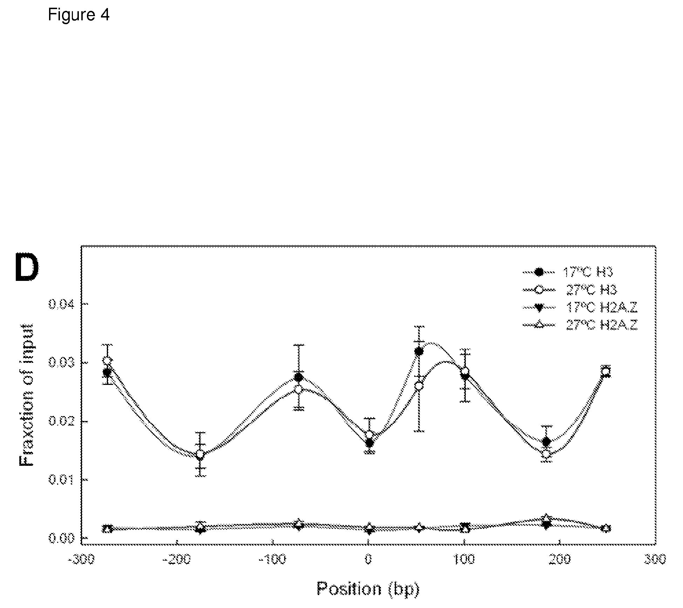

### Figure 4. H2A.Z occupancy varies as a function of ambient temperature

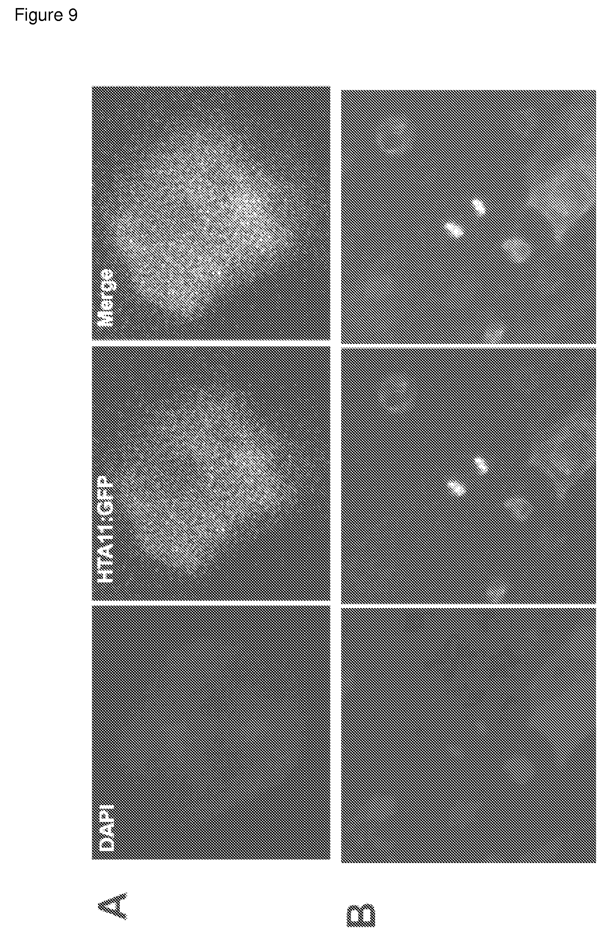

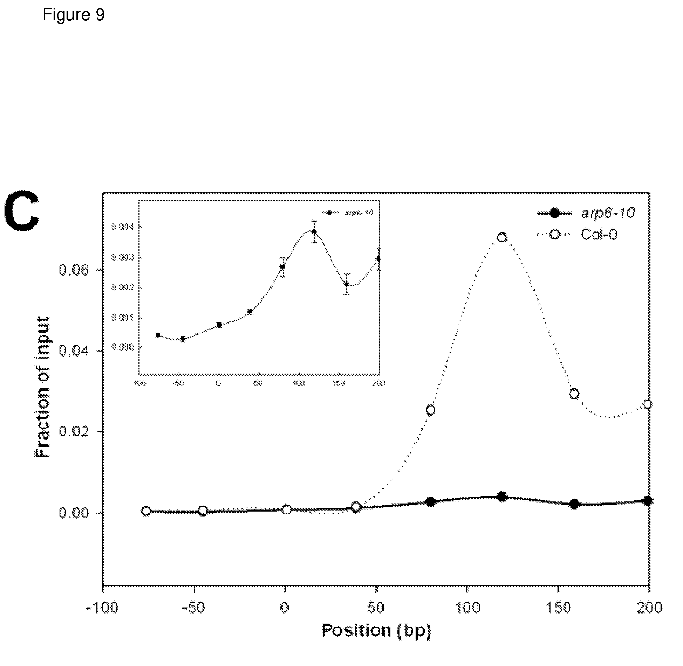

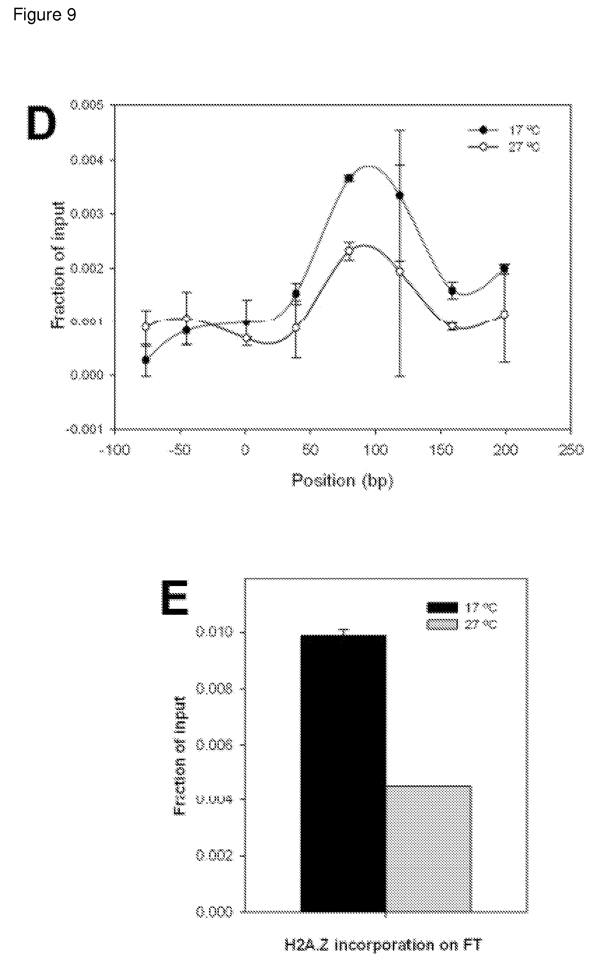

(A) *HTA11:GFP* expressed under the native promoter is functional and complements the *hta9 hta11* double mutant. The *hta9 hta11* mutant has an extended hypocotyl compared to Col-0 at 21 °C. This phenotype was completely rescued in three independent lines expressing *HTA11:GFP* under the native promoter (left panel). *HTA11:GFP* also complements the elevated *HSP70* expression in the *hta9 hta11* double mutant (right panel).
(B) Enrichment of H2A.Z at the *HSP70* promoter was analysed using *HTA11:GFP.* Formaldehyde cross-linked chromatin from seven day old seedlings grown at 17 °C (open circles) or after 2 hours of incubation at 27 °C (closed circles) were digested with MNase and were used for chromatin immunopurification (ChIP) analyses. H2A.Z immuno-complex associated DNA fragments were quantitatively analysed using tiling oligonucleotides (See Figure 4B). A region corresponding to the +1 nucleosome is enriched in H2A.Z where as the -1 nucleosome has a much reduced H2A.Z occupancy (inset bar graph, 4A). x-axis: the nucleotide positions across *HSP70* gene with respect to TSS. y-axis: H2A.Z occupancy as a fraction of undigested input chromatin. Values are mean ± SD from one representative experiment. Bar graph in the inset shows the -1 nucleosome in detail as represented by the amplicon centred on -246 bp.
(C) ChIP analysis of cross-linked H3 on seedlings treated similarly as above shows the well positioned nucleosomes of *HSP70* promoter. On temperature shift to 27 °C, H3 occupancy changed at the +1 nucleosome where H2A.Z occupancy is high. The relative change in H3 occupancy in response to temperature shift was lower than that of H2A.Z.
(D) A gypsy like transposon gene (At4g07700) acts as a control since it has positioned nucleosomes devoid of H2A.Z and it is transcriptionally inactive and unresponsive to temperature changes. ChIP analysis revealed no change in histone H3 occupancy at both 17 °C and 27 °C, indicating that H2A.Z confers temperature specific responsiveness to nucleosomes. As expected, H2A.Z occupancy was absent as measured by ChIP. See also Figure 9.

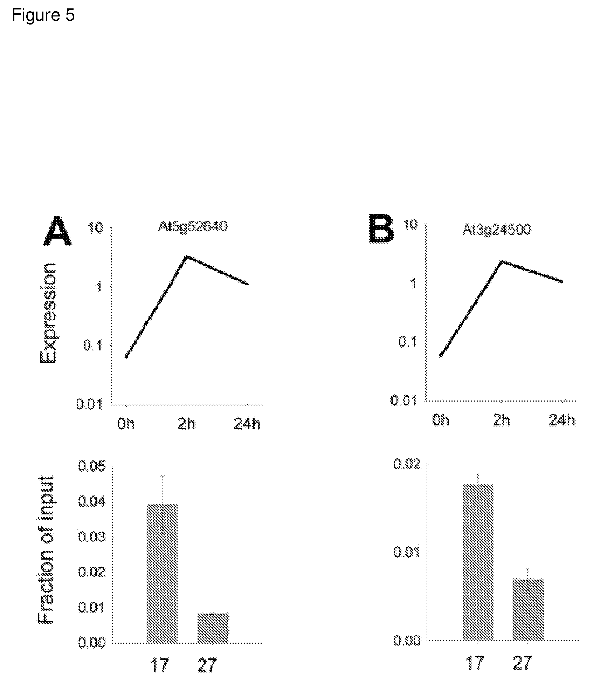

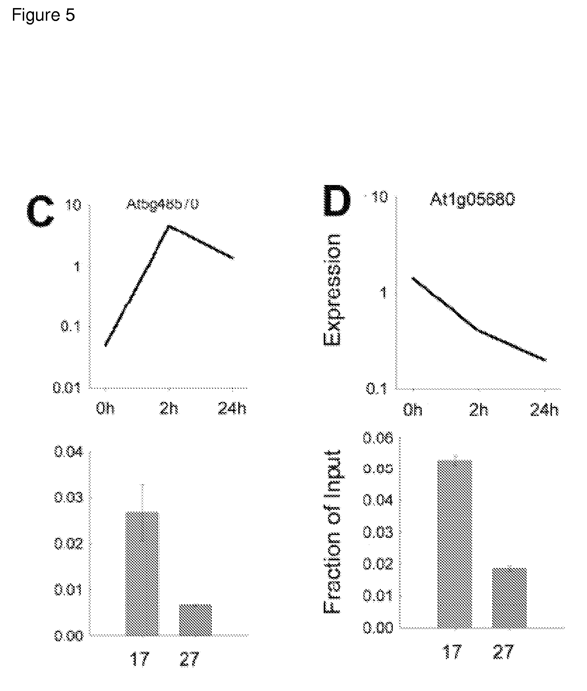

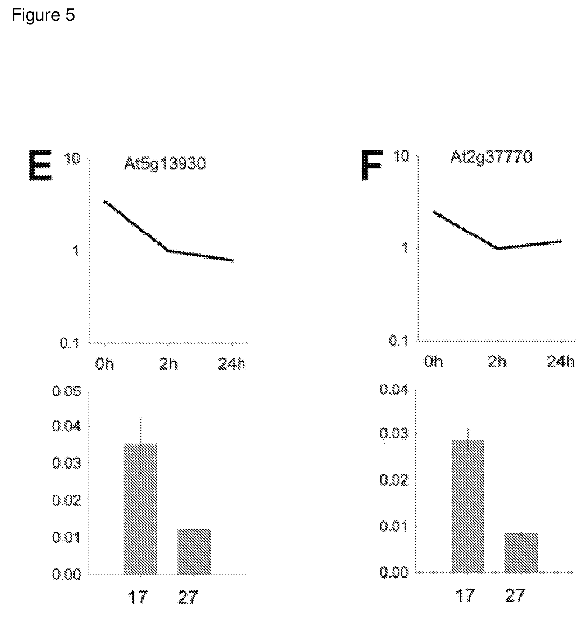

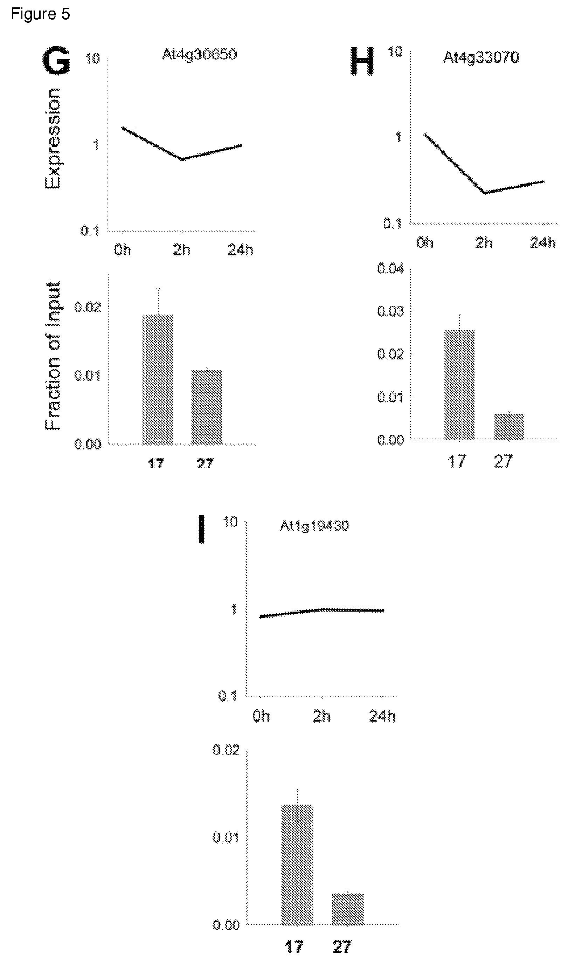

### Figure 5. H2A.Z occupancy dynamics are independent of the transcriptional response.

(A - I) Genes with diverse transcriptional response were analysed for H2A.Z occupancy in response to temperature shift. H2A.Z occupancy is primarily determined by temperature, independent of the specific transcriptional response. This result indicates that H2A.Z containing nucleosomes confer temperature information on chromatin, which could be interpreted according to the respective regulatory environment for appropriate gene expression. Upper panels show the transcriptional response upon shift to 27 °C. 0 h represents sample from plants grown at 12 °C; 2h and 24 h are after shift to 27 °C. H2A.Z occupancy dynamics upon shift to 27°C for 2h is shown in the lower panel.

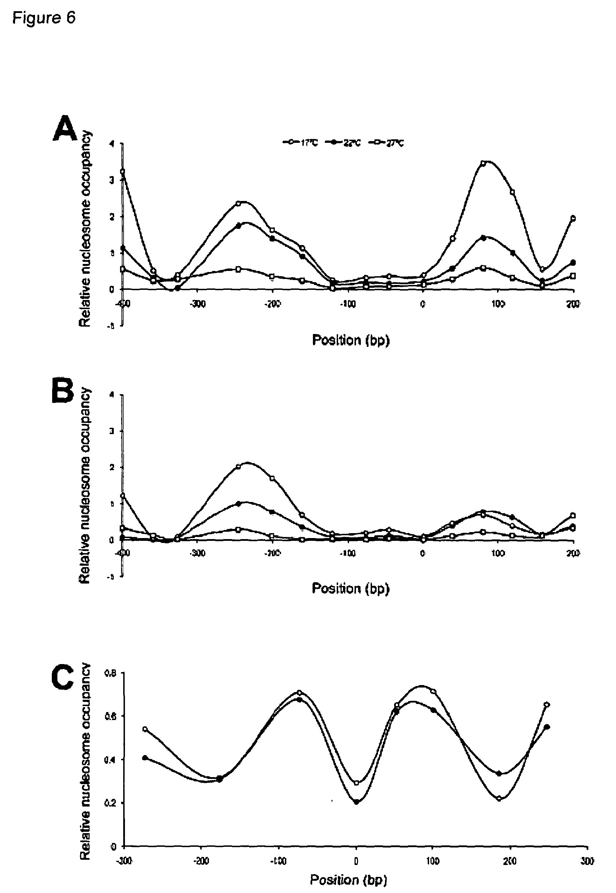

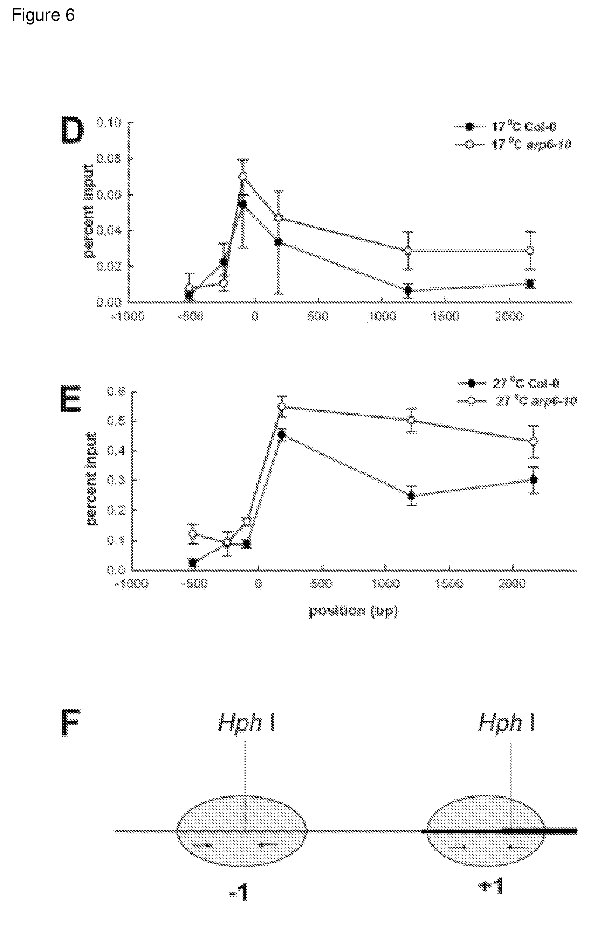

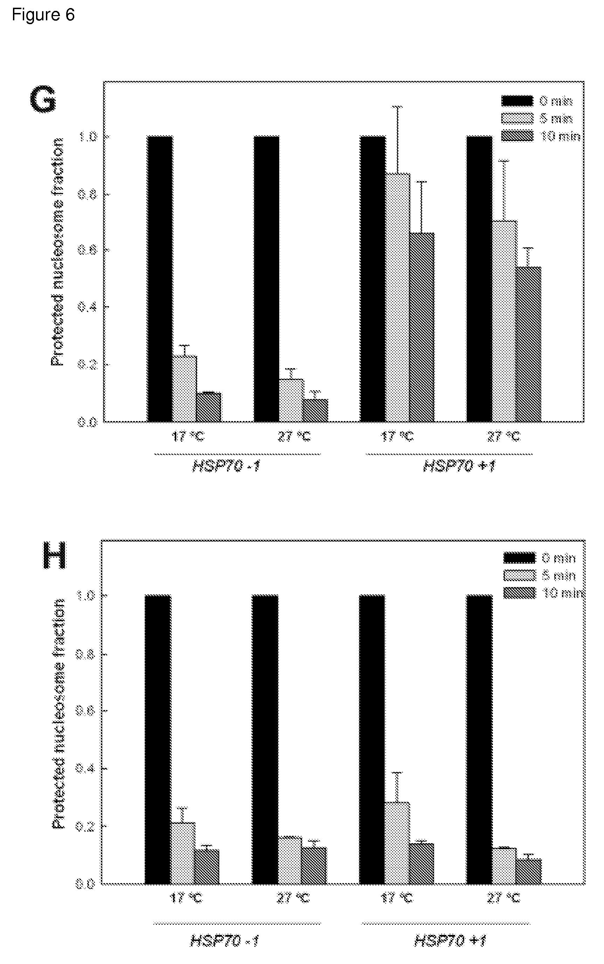

### Figure 6. Chromatin architecture responds dynamically to changes in ambient temperature.

(A) Chromatin architecture dynamics at *HSP70* in response to temperature in wild-type. Chromatin from seven day old seedlings growing at 17 °C (open circle), 22 °C (closed circle) and 27 °C (square) was MNase treated and analysed by Q-PCR with tiled oligos. The +1 nucleosome of *HSP70* exhibits a robust response to increasing ambient temperature, with nucleosome occupancy and temperature being negatively correlated.
(B) *arp6* shows a constitutive temperature response in terms of chromatin architecture as measured by MNase accessibility. +1 nucleosome occupancy in *arp6* is consistently lower, even at 17 °C.
(C) Analysis of nucleosome occupancy dynamics in response to temperature at At4g07700 reveals that consistent with the H3 data, nucleosomes at this locus are non-responsive to changes in ambient temperature.

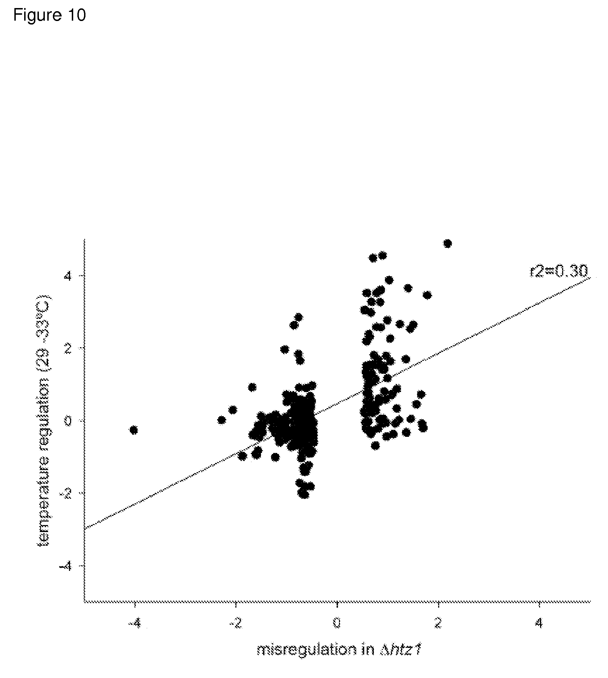

ChIP analysis for RNA Pol II at *HSP70* in wild-type (closed circle) and *arp6* (open circle) grown at 17 °C (D) or after 2h of incubation at 27 °C (E). x-axis represents nucleotide positions on *HSP70* with respect to TSS; y-axis represents fraction of input chromatin immunopurified using RNA Pol II antibody.
(F) Schematic representation of HSP70 +1 and -1 nucleosomes showing the *Hph* I sites. Grey ovals represent nucleosomes, oligo positions are indicated by arrows. (G-H) Temperature-dependent nucleosomal DNA accessibility assay for *Hph* I enzyme in wild-type (G) and *arp6*-*10* (H). Nucleosomes containing H2A nucleosomes (-1 in wild-type and both -1 and +1 in *arp6*-*10*) show constitutive accessibility of restriction sites, whereas H2A.Z containing nucleosomes occlude access. Relative amounts of protected input nucleosomal DNA was calculated and was normalised against the +1 nucleosome of At4g07700 where *Hph* I does not cut. See also Figure 10.

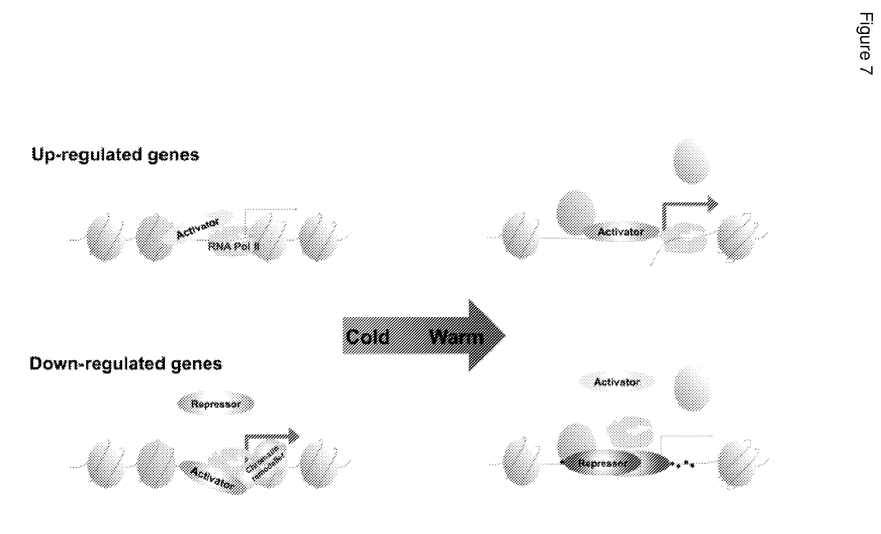

### Figure 7. H2A.Z containing nucleosomes can modulate transcription in a temperature dependent manner.

At lower temperature, H2A.Z nucleosomes have a high level of occupancy. H2A.Z nucleosomes may prevent transcription, either by acting as a physical block to the progression of RNA Pol II, or by occluding gene specific cis-elements from activating transcription factors. For genes that are specifically expressed at low temperature, H2A.Z occupancy may prevent the binding of repressors or antagonise DNA methylation. A chromatin-remodelling complex may relieve the nucleosomal repression when appropriate components and conditions exist. At higher temperature, H2A.Z nucleosome occupancy declines. This leads to increased expression of genes like *HSP70,* where transcription is limited by H2A.Z occupancy. For genes whose expression is decreased at higher temperature, this loss of H2A.Z may facilitate repressor binding. Conventional H2A nucleosomes are depicted in light gray ovals and the degree of H2A.Z occupancy is depicted in darker gray.

### Figure 8. Temperature induced architectural phenotypes are constitutive in arp6-10

(A) Hypocotyl elongation was quantified from 15-day-old seedlings of WT and *arp6*-*10* grown at respective temperatures were quantified. (B-D) Petiole elongation in first four leaves of 20-day-old seedlings of WT and *arp6-10* grown at 17 °C (B), 22 °C (C) and 27 °C (C) were quantified.

### Figure 9. HTA11:GFP fusion protein is localized and functions as the endogenous protein

Confocal microscopy reaveals the nuclear distribution of HTA11:GFP in the nucleus and exclusion from the heterochromatic regions (A) consistent with the reported pattern of the endogenous protein (Deal et al., 2007, Zilberman et al., 2008). (B) Visualisation of an anaphase cell shows the incorporation of HTA11:GFP into chromosomes. (C) Chromatin immunoprecipitation analysis of MNase digested chromatin from *arp6*-*10* shows the drastic reduction of H2A.Z deposition into +1 nucleosome of *HSP70.* H2A.Z occupancy profile of wild type Col-0 is shown as dotted line for reference. Insert shows the *arp6*-*10* profile alone. (D) Chromatin from 17 °C grown seedlings as well as from those shifted to 27 °C for 2h were digested with MNase and were used for ChIP experiments using antibody against H2A.Z showing temperature responsive H2A.Z dynamics. These results further validate the HTA11:GFP protein fusion and our ChIP results using the same. (E) ChIP for H2A.Z occupancy at the TSS proximal region of FT, a key regulator of the thermal induction of flowering. Consistent with the other results we found that H2A.Z occupancy is at this locus is a function of ambient temperature temperature.

### Figure 10. HTZ1 regulates by temperature regulon in yeast.

Expression of genes assigned to be up- and down-regulated in the *htz1Δ* background (y-axis) (Meneghini et al., 2003) was compared with gene expression on shifting from 29 °C to 33 °C (x-axis) (Gasch et al., 2000). There is a strong correlation: genes constitutively down-regulated in the *htz1Δ* background tend to be those genes whose expression is inhibited on increasing temperature, while those genes whose expression goes up at 33 °C tend to be more highly expressed in the *htz1Δ* mutant at 30 °C. These results are consistent with a role for Htz1 containing nucleosomes to provide thermal information for regulating the temperature transcriptome. Temperature induced loss of Htz1 from chromatin at higher temperatures may contribute to the regulation of temperature responsive genes (R² 0.30, P < 0.001).

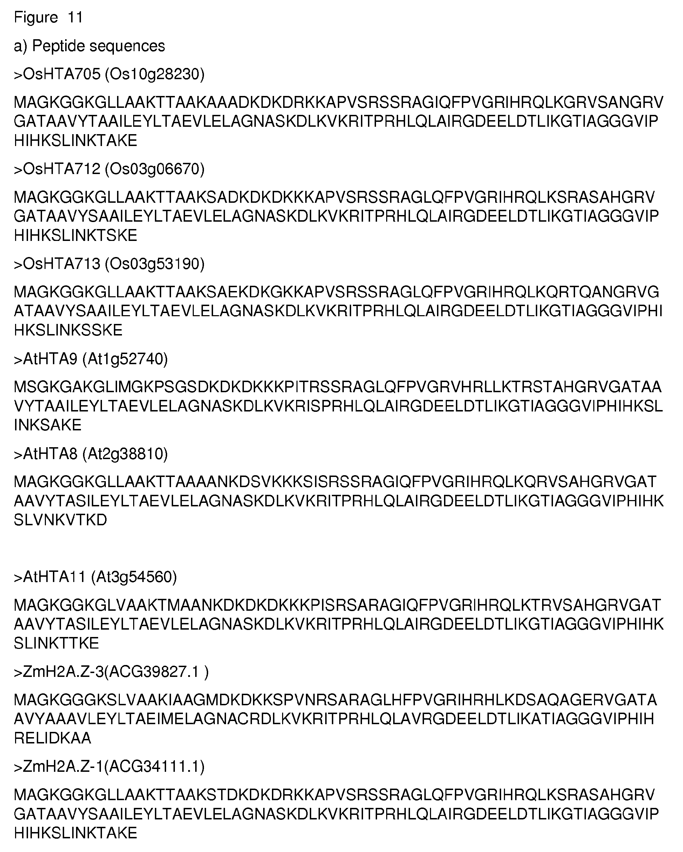

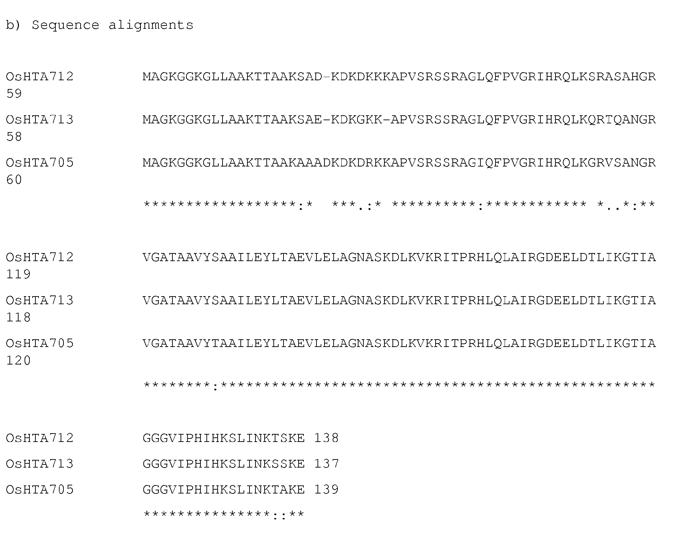

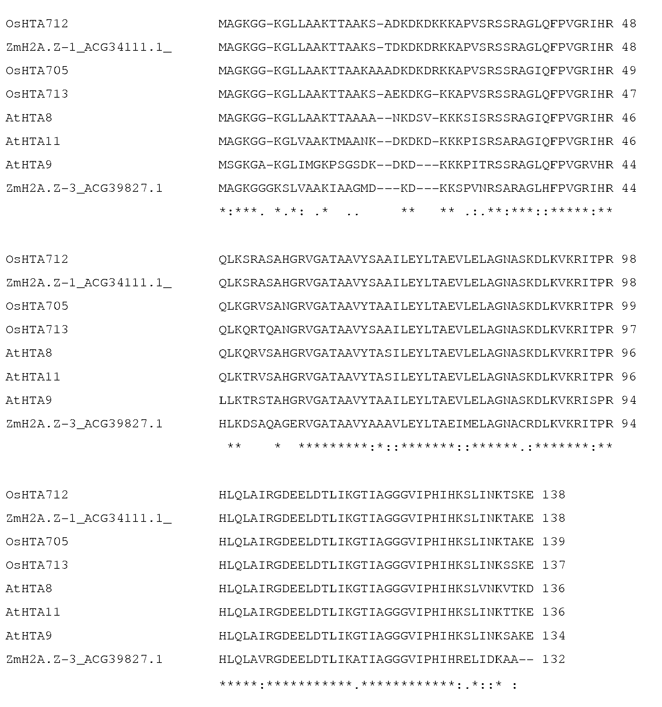

### Figure 11. Peptide sequences encoding H2A.Z in Arabidopsis, rice and maize and sequence alignment

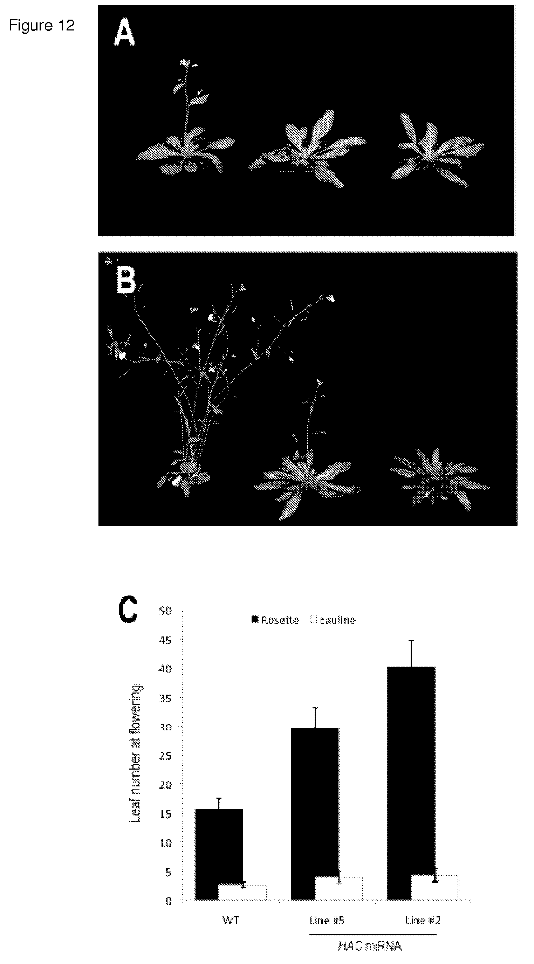

### Figure 12. Flowering data from HAC miRNA lines

Over-expression of an artificial microRNA targeting the HAC genes in Arabidopsis results in delayed flowering. Two representative stable transgenic lines #5 and #2 are shown with the wildtype at two different time points, one at the time the wildtype flowers (A) and many weeks later (B) to show the extend of delayed flowering. The bar graph (C) shows the flowering data in terms of leaf number at the time of flowering. Dark bars represent rosette leaves and grey bar represent cauline leaves.

### DETAILED DISCLOSURE OF THE INVENTION

The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

The practice of the present invention will employ, unless otherwise indicated, conventional techniques of botany, microbiology, tissue culture, molecular biology, chemistry, biochemistry and recombinant DNA technology, which are within the skill of the art. Such techniques are explained fully in the literature.

Plants are highly sensitive to temperature and can perceive a difference of as little as 1 °C. How temperature is sensed and integrated in development is largely unknown. We have found that the presence or absence of the alternative histone H2A.Z in the nucleosome is essential for how a plant senses temperature and temperature changes. Surprisingly, we have found that the presence or absence of the alternative histone H2A.Z in the nucleosome is an essential central point of regulation in the perception of temperature and thus the thermosensory response, which includes regulation of the expression of a large number of temperature dependent genes (the temperature transcriptome). Thus, although temperature sensing and eliciting temperature-dependent responses is known to be a complex biological process, the inventors have shown that a central single regulator in this mechanism is H2A.Z. Moreover, the inventors have shown that by modifying the presence or absence of H2A.Z in the nucleosome, it is possible to regulate the ways in which a plant perceives temperature and thus to alter developmental response that are dependent on temperature. It is therefore possible to alter a large variety of temperature dependent responses, including developmental responses in plants such as flowering, hypocotyl elongation and petiole growth, by targeting this single regulator.

Thus, we describe methods for altering temperature sensing in a eukaryotic organism, for example in a plant, and thus modifying thermosensory responses by altering the presence of H2A.Z in the nucleosome.

As shown herein, the presence of H2A.Z in the nucleosome is rate limiting for the expression of genes that are dependent on increasing temperature. Thus, in a wild type organism, H2A.Z is present in the nucleosome at cold temperatures. H2A.Z responds to an increase in the ambient temperature and is released from the nucleosome. This release induces the expression of temperature dependent genes that are expressed at higher temperatures, whilst cold temperature dependent genes are no longer expressed. H2A.Z thus functions as a temperature sensor and the presence or absence of this regulator determines gene expression which in turn regulates developmental responses that are normally induced by changes in temperature. As discussed in the examples, when H2A.Z deposition is prevented, plants display a constitutive warm temperature response even in cold temperature, i.e. a response that the plant would normally display in warm temperature.

As shown herein, genotypes deficient in incorporating H2A.Z into nucleosomes phenocopy warm grown plants even when grown at lower temperatures. As a skilled person will appreciate responses to changes in temperature vary between different plants, depending on the specific temperature threshold of the plant. However, generally speaking, by warm grown plants is meant plants grown at a temperature of about 20 °C to about 24 °C, for example about 22 °C or above for Arabidopsis. By cold temperature is meant plants gown at a temperature of about 15 °C to about 19 °C, for example about 17 °C or below for Arabidopsis. Other plants have different temperature optima.

Genotypes deficient in incorporating H2A.Z into nucleosomes also show altered gene expression and the gene expression profile is that typically shown by plants grown in warm temperature conditions. Thus, nucleosomes containing H2A.Z display distinct responses to temperature *in vivo,* independently of transcription. Using purified nucleosomes, we are able to show that H2A.Z confers distinct DNA unwrapping properties on nucleosomes, indicating a direct mechanism for the perception of temperature through DNA-nucleosome fluctuations. Our results show that H2A.Z-containing nucleosomes provide thermosensory information that is used to coordinate developmental responses and the ambient temperature transcriptome. We observe the same effect in budding yeast, indicating this is an evolutionarily conserved mechanism.

In a first aspect, the invention relates to a method for altering temperature sensing in a plant or a part thereof comprising modifying the presence or state of H2A.Z in the nucleosome by decreasing or increasing the level of H2A.Z in said plant or a part thereof or altering post-translational modification of H2A.Z, wherein said post-translational modification is acetylation.

We also describe a method for altering thermosensory responses in a eukaryotic organism said method comprising decreasing the level of H2A.Z in said organism or altering post-translational modification of H2A.Z or a combination thereof.

The eukaryotic organism according to the different methods as described in this disclosure may be a plant, a yeast, a fungus, an invertebrate or a vertebrate. In one embodiment, the organism is a plant. The organism may also be an isolated part of a plant, such as a plant cell, for example a plant cell culture or cell line. Thus, in one aspect, the invention relates to a method for altering temperature sensing in a plant or a part thereof comprising modifying the presence or state of H2A.Z in the nucleosome by decreasing or increasing the level of H2A.Z in said plant or a part thereof or altering post-translational modification of H2A.Z, wherein said post-translational modification is acetylation.

Where the organism is a plant, it may be a moncot or a dicot.

A dicot plant may be selected from the families including, but not limited to *Asteraceae, Brassicaceae (eg Brassica napus), Chenopodiaceae, Cucurbitaceae, Leguminosae (Caesalpiniaceae, Aesalpiniaceae Mimosaceae, Papilionaceae or Fabaceae), Malvaceae, Rosaceae or Solanaceae.* For example, the plant may be selected from lettuce, sunflower, *Arabidopsis,* broccoli, spinach, water melon, squash, cabbage, tomato, potato, yam, capsicum, tobacco, cotton, okra, apple, rose, strawberry, alfalfa, bean, soybean, field (fava) bean, pea, lentil, peanut, chickpea, apricots, pears, peach, grape vine or citrus species. In one embodiment, the plant is oilseed rape.

Also included are biofuel and bioenergy crops such as rape/canola, linseed, lupin and willow, poplar, poplar hybrids, Miscanthus or gymnosperms, such as loblolly pine. Also included are crops for silage (maize), grazing or fodder (grasses, clover, sanfoin, alfalfa), fibres (e.g. cotton, flax), building materials (e.g. pine, oak), pulping (e.g. poplar), feeder stocks for the chemical industry (e.g. high erucic acid oil seed rape, linseed) and for amenity purposes (e.g. turf grasses for golf courses), ornamentals for public and private gardens (e.g. snapdragon, petunia, roses, geranium, Nicotiana sp.) and plants and cut flowers for the home (African violets, Begonias, chrysanthemums, geraniums, Coleus spider plants, Dracaena, rubber plant).

A monocot plant may, for example, be selected from the families *Arecaceae, Amaryllidaceae* or Poaceae. For example, the plant may be a cereal crop, such as wheat, rice, barley, maize, oat sorghum, rye, onion, leek, millet, yam, buckwheat, turf grass, Italian rye grass, sugarcane or Festuca species.

Preferably, the plant is a crop plant. By crop plant is meant any plant which is grown on a commercial scale for human or animal consumption or use.

Preferred plants are maize, wheat, rice, oilseed rape, sorghum, soybean, potato, tomato, grape, barley, pea, bean, field bean, lettuce, cotton, sugar cane, sugar beet, broccoli or other vegetable brassicas or poplar.

In other embodiments of this disclosure, the organism is a yeast or fungus. For example, many of processes mediated in the brewing, baking and fermenting industries are very dependent on temperature responses of the cultured yeast strains. Since the H2A.Z nucleosome eviction pathway is extremely highly conserved across the eukaryotes, this provides a means to alter the responses of commercially important yeasts to temperature in a targeted fashion using the methods described herein.

The different ways in which the method for altering temperature sensing and the method for altering thermosensory response are envisaged are set out below.

According to methods of the invention, the presence of H2A.Z in the nucleosome can be modified in a number of ways. These include altering, decreasing or inhibiting expression of genes that encode H2A.Z. Various methods known to a skilled person can be used to achieve this, for example using RNA interference (siRNA) technology. Alternatively, the presence of H2A.Z in the nucleosome can be modified by altering the status of H2A.Z, for example the post-translational modification status of H2A.Z. This includes post-translational modifications such as acetylation, ubiquitination and methylation. The presence of H2A.Z in the nucleosome can also be modified by altering assembly of H2A.Z into the nucleosome or eviction of H2A.Z from the nucleosome.

Thus, in one embodiment of methods of the invention, the invention relates to modifying the presence of H2A.Z in the nucleosome by decreasing the level of H2A.Z peptide or protein in the plant said method comprising decreasing or inhibiting the expression of one or more nucleic acids encoding for H2A.Z. According to this embodiment, the level or amount of H2A.Z peptide or protein is reduced, for example so that substantially no H2A.Z peptide or protein is produced in said organism. Thus, no or little H2A.Z can be incorporated into the nucleosome. The reduced level or substantial lack of H2A.Z in the nucleosome influences temperature sensing and transcriptional control of genes that respond to changes in temperature and therefore temperature dependent developmental processes. This leads to the induction of a constitutive warm temperature response even in cold temperature conditions.

As a skilled person will appreciate, different methods known in the art can be employed to decrease or inhibit the expression of one or more nucleic acids encoding for H2A.Z according to the methods of the invention. For example, said nucleic acid(s) may be silenced using RNA interference. This comprises for example expression of a simple antisense RNA, hairpin RNA, a natural or artificial microRNA or other RNA interference methods known in the art. Plants with reduced or no expression of H2A.Z encoding nucleic acid(s) may also be selected from a mutagenised population through Targeting-induced Local Lesions IN Genomes (TILLING)(Till et al., 2006).

H2A.Z is universally conserved in eukaryotic organisms and has been identified in mammals, birds, Drosophila, yeast, tetrahymenea and plants.

In plants, there are several nucleic acids that encode H2A.Z. For example, in Arabidopsis, the four H2A.Z genes are *HTA8* (At2g38810), *HTA9* (At1g52740), *HTA4* (At4g13570) and *HTA11* (At3g54560).

H2A.Z has been identified in different plants and nucleic acid sequences encoding H2A.Z can be accessed on public databases. Plant H2A.Zs are very well conserved across different taxa, including algae, maize, mosses, rice and sorghum. H2A.Z sequences from algae, mosses, rice, sorghum have conserved N-terminal acetylatable lysines (K) as in Arabidopsis. A comparison of the peptide sequences encoding H2A.Z in Arabidopsis, rice and maize shows that these sequences are highly conserved (see figure 11). Thus, a skilled person would be able to modify any plant H2A.Z according to the methods disclosed herein.

In another embodiment of methods of the invention, post-translational modification of H2A.Z is modified to alter the presence of H2A.Z in the nucleosome and/or alter thermosensory responses in the organism. This can for example be achieved as set out below.

For example, the extent to which H2A.Z is acetylated can be modified. Acetylation reversibly modifies histones and thus chromatin structure. Acetylated chromatin is less tightly folded and therefore more accessible to interacting proteins. Site specific acetylation leads to activation of transcription whereas deacetylation represses transcription. Hyper-acetylated histone loosens contact with DNA, thus making nucleosome core particles less rigid.

Histone tails are normally positively charged due to amine groups present on their lysine (K) and arginine (R) amino acid residues. These positive charges help the histone tails to interact with and bind to the negatively charged phosphate groups on the DNA backbone. Acetylation of lysine (K), which occurs normally in a cell, neutralizes the positive charges on the histone by changing amines into amides and decreases the ability of the histones to bind to DNA. This decreased binding allows chromatin expansion, permitting transcription to take place.

The acetylation of histones is regulated by histone acetyltransferase enzymes (HAT). HATs are enzymes that acetylate conserved lysine amino acids on histone proteins by transferring an acetyl group from acetyl CoA to form ε-N-acetyl lysine. Acetylation can be reversed by histone deacetylation (HDAC) enzymes. HDACs remove acetyl groups, increasing the positive charge of histone tails and encouraging high-affinity binding between the histones and DNA backbone. The increased DNA binding condenses DNA structure, preventing transcription. Acetylation occurs predominantly in the highly conserved N-terminus of histones and at lysine (K) residues. H2A.Z sequence is highly conserved amongst eukaryotic organisms and comprises a number of acetylation sites. Arabidopsis H2A.Z proteins contain at least 4 to 5 potentially acetylatable K residues in its N-terminal tail, a feature that is conserved across different H2A.Z sequences across different plant species. Thus, according to the methods of the invention, the conserved acetylatable K residues in the N-terminal tail of plant H2A.Z proteins can be targeted.

According to the methods of the invention, one or more acetylation sites of H2A.Z can be targeted to decrease or increase the degree of acetylation or to substantially prevent acetylation of H2A.Z thereby altering the presence of H2A.Z in the nucleosome and/or altering thermosensory responses in the organism.

For example, site-directed mutagenesis of a gene sequence encoding for a H2A.Z peptide or protein can be used to change one or all of the K residues to a non-acetylatable residue (H2A.Z^{N}). For example, the non-acetylatable residue may be arginine (R) or another amino acid residue.

Mutagenesis of acetylatable K residues reduces or prevent acetylation. Reduced acetylation sites prevent the access of HATs, subsequent acetylation, transformation of chromatin into a less rigid structure and transcription of genes that are transcribed at elevated temperature conditions. Thus, reducing acetylation sites produces a H2A.Z^{N} that has a high affinity for DNA, and will not be evicted from the nucleosome at higher temperatures, preventing the transcription of genes that would normally be transcribed at higher temperatures and therefore developmental responses that would normally occur in response to higher temperatures.

In another embodiment, a gene sequence encoding a H2A.Z peptide or protein can be modified to introduce further acetylation sites resulting in a hyper-acetylated H2A.Z protein. This facilitates release of H2A.Z from the nucleosome. H2A.Z absence from the nucleosome results in a constitutive warm temperature response even at colder temperature, as the transcription of genes that are responsive to higher temperature is induced whilst the transcription of genes that are responsive to cold temperature is repressed.

The altered gene sequences described above can be expressed in the organism using expression vectors commonly known in the art. The mutated sequence may be part of an expression cassette comprising a promoter driving expression of said sequence. Said promoter may be the endogenous promoter, a constitutive promoter, or a tissue specific promoter. Using a tissue specific promoter, it is possible to drive expression of the transgene in a tissue specific way thus altering temperature sensing in a particular tissue. In plants, the tissue specific promoter may for example be selected from the TaPR60 Promoter that has been demonstrated to be specifically active in endosperm transfer cells in wheat and barley and starchy endosperm in rice (Kovalchuk et al., 2009), the OsPR602 and OsPR9a promoters from rice which are specific for endosperm transfer cells (Li et al., 2008), the granule-bound starch synthase 1 (gbss1) promoter which is specific to the grain filling process in wheat (Kluth et al., 2002), the napA promoter from rape for seed specific expression in crucifers, the *E4, E8* or *2a11* promoter from tomato or the *AGPL1* promoter from water melon for fruit specific expression and the TA29 promoter from tobacco or the A9 promoter from Arabidopsis for anther/tapetum specific expression.

Overexpression using a constitutive promoter in plants may be carried out using a strong promoter, such as the cauliflower mosaic virus promoter (CaMV35S), the rice actin promoter, the maize ubiquitin promoter, the rice ubiquitin rubi3 promoter or any promoter that gives enhanced expression. Alternatively, enhanced or increased expression can be achieved by using transcription or translation enhancers or activators and may incorporate enhancers into the gene to further increase expression. Furthermore, an inducible expression system may be used, such as a steroid or ethanol inducible expression system in plants.

In another embodiment of methods of the invention, enzymes that acetylate or de-acetylate H2A.Z are targeted to alter the presence of H2A.Z in the nucleosome and/or alter thermosensory responses in the plant according to methods of the invention.

In one embodiment of methods of the invention, acetylation of the H2A.Z peptide or protein may be decreased or inhibited by decreasing or inhibiting HAT activity. For example, one or more nucleic acid sequence that encodes a HAT enzyme may be silenced using RNA interference. A construct directed against a conserved region of the HAT family may be used to decrease the levels of HAT transcript. Also, HAT activity may be decreased or inhibited by altering a nucleic acid sequence that encodes a HAT enzyme by site directed mutagenesis to decrease activity of the enzyme. Plants with reduced or little expression of HAT encoding nucleic acid(s) and/or mutated HAT protein(s) with decreased or no enzyme activity may be selected from a mutagenised population through TILLING (Till et al., 2006).

This will reduce the amount of acetylation of H2A.Z and thus prevent eviction of H2A.Z from the nucleosome. This leads to a decrease of the expression of genes requiring H2A.Z eviction from the nucleosome for their expression and thus a decrease or inhibition of developmental responses that would normally be induced by high temperature.

In another embodiment, expression of nucleic acid sequence that encodes a HAT enzyme is increased, for example by introducing into said organism one or more nucleic acids encoding HAT or otherwise altering HAT activity, for example by mutagenesis of one or more nucleic acid sequence encoding HAT. The expression of the nucleic acid sequence may be driven by the endogenous, constitutive or a tissue specific promoter. This will increase the amount of acetylation and thus increase release of H2A.Z from the nucleosome.

In another embodiment, the acetylation status of the H2A.Z peptide or protein in the nucleosome may be increased by decreasing or inhibiting HDAC activity. Decreasing or inhibiting HDAC activity prevents or reduces the removal of the acetyl group from H2A.Z. This results in reduced binding between H2A.Z and DNA and thus release of H2A.Z from the nucleosome and thus expression of genes that respond to high temperatures.

According to another embodiment of this aspect of the invention, histone deacetylase (HDAC) enzymes are expressed at enhanced levels or HDAC activity is otherwise altered, for example by mutagenesis of one or more nucleic acids encoding HAT. This prevents release of the H2A.Z from the nucleosome from the nucleosome as acetyl groups are removed. For example, one or more genes encoding for a HDAC enzyme may be constitutively expressed in the organism. A tissue specific promoter may be used.

H2A.Z is assembled into the SWR1 nucleosome by the chromatin re-modelling machinery. The components of this complex have been shown to be highly conserved amongst eukaryotes. For example, ARP6 in Arabidopsis has been shown to be responsible for inserting H2A.Z into the nucleosome in plants and is part of the SWR-1 complex. Depletion of ARP6 in mutants results in the failure to incorporate H2A.Z into chromatin and constitutive expression of the warm temperature transcriptome.

Several plant ARP6 homologues have been identified and are highly evolutionarily conserved across the plant kingdom. For example, analysis of the respective nucleic acid sequences for ARP6 homologue of rice, maize, capsella, and mosses reveals that these are highly similar to that of Arabidopsis indicating conservation across plants. Thus, a skilled person would be able to identify and modify an ARP6 homologue according to the methods of the invention.

By modifying components of the chromatin re-modelling machinery, for example a histone chaperone, the insertion or assembly of H2A.Z into the nucleosome can be prevented. Thus, in one embodiment of the methods of the invention, the presence of H2A.Z in the nucleosome can be altered by modifying the components of the chromatin re-modelling machinery. For example, in one embodiment, expression of one or more genes that encode one or more proteins involved in the insertion of H2A.Z into the nucleosome can be inhibited, for example by RNA interference. This leads to a lack of H2A.Z in the nucleosome and thus a constitutive warm temperature response. Factors that may be targeted include histone chaperones and members of the SWR-1 complex. For example, in plants ARP6 or a homologue or orthologue thereof may be targeted. Other targets may include histone chaperones that promote assembly, such as NAP1, CHZ1 or a homologue or orthologue thereof.

In another embodiment of methods of the invention, components of the chromatin remodelling machinery, for example a histone chaperone, may be targeted to prevent disassembly of H2A.Z from the nucleosome. For example, genes that encode such factors may be silenced.

As mentioned elsewhere, the presence or absence of H2A.Z in the nucleosome induces thermosensory responses. As stated above, in one aspect, the invention therefore relates to a method for altering thermosensory responses in a plant, said method comprising decreasing the level of H2A.Z in said plant or altering post-translational modification of H2A.Z or a combination thereof.

The ways of decreasing the level of H2A.Z in said plant or altering post-translational modification of H2A.Z are explained in detail above and apply to the different embodiments of this aspect of the invention. For example, the expression of one or more nucleic acids encoding for H2A.Z may be decreased or inhibited as explained above. For example, said nucleic acid is silenced using RNA interference.

In another embodiment, post-translational modification such as acetylation may be changed as explained above. For example, site-directed mutagenesis of a gene sequence encoding for a H2A.Z peptide or protein can be used to change one or all of the K residues to a non-acetylatable residue (H2A.Z^{N}) or to introduce further acetylation sites. Moreover, as detailed above, the expression of one or more nucleic acid sequence encoding HDACs or HATs may be decreased, inhibited or enhanced to alter thermosensory responses.

For example, a mutant plant H2A.Z protein may be produced by expressing a nucleic acid sequence encoding a H2A.Z protein wherein said nucleic acid sequence has been altered to remove one or more acetylation sites, for example by altering a codon encoding an acetylatable K residue in said nucleic acid sequence by replacing or altering a nucleotide within said codon to produce a protein with non acetylatable residue. In another embodiment, a mutant H2A.Z protein may be produced by expressing a nucleic acid sequence encoding for a H2A.Z protein wherein said nucleic acid sequence has been altered to introduce one or more additional acetylation sites. As explained elsewhere, promoters according to the invention may be either the endogenous, a constitutive or a tissue specific promoter.

Altering post-translational modification according to the method may also comprise inhibiting or decreasing the expression of one or more nucleic acid sequence encoding for a HDAC enzyme to reduce or inhibit deacetylation of H2A.Z or expressing one or more nucleic acid sequences encoding a HDAC enzyme to increase removal of acetyl groups in H2A.Z.

In another embodiment, altering post-translational modification according to the method may also comprise inhibiting or decreasing the expression of one or more genes encoding for a HAT enzyme to inhibit acetylation or expressing one or more nucleic acid sequences encoding a HAT enzyme to increase acetylation of H2A.Z.

Expression of said nucleic acid sequences according to the method may be driven by the endogenous, a constitutive, inducible or a tissue specific promoter.

Thermosensory responses are those responses that are observed in an organism in response to changes in temperature. These include changes in gene transcription. In plants, genes such as HSP70 are up-regulated at higher temperatures. HSP70 gene transcription follows a proportionate pattern with increasing temperature over a wider range (about 12°C to ≥37 °C) changes in gene transcription (of the temperature transcriptome) and can therefore be used as a marker gene which indicates up or down-regulation of the temperature transcriptome. Transcriptional changes also influence developmental processes and in plants an increase of temperature affects a number of developmental processes, such as flowering, hypocotyl elongation and petiole growth. It also leads to an increase in the rate of transition through different developmental phases. Juvenile to adult transition is therefore accelerated by higher ambient temperature. An increase in temperature leads to acceleration of flowering time, greater hypocotyl elongation, petiole growth, germination, and general acceleration of plant growth and development.

According to the methods of the invention, the thermosensory responses in a plant that can be altered by the method described above and said responses include one or more of and preferably all of the following developmental responses: seed dormancy release, germination, hypocotyl elongation, petiole growth, vernalisation, juvenile to adult transition, flowering, senescence and temperature-protective responses.

In one embodiment, the entirety or totality of the thermosensory response in a plant is modified. Thus, substantially all of the developmental and transcriptional responses that a plant shows in response to changes in temperature can be altered by increasing or decreasing the level of H2A.Z in said organism or altering post-translational modification of H2A.Z or a combination thereof.

Another aspect of the disclosure relates to a method for inducing a warm temperature response in a eukaryotic organism comprising inhibiting or decreasing the presence H2A.Z in the nucleosome. The response may be constitutive and is induced even in temperatures which would normally be perceived as cold temperatures by said plant. The method may include modifying the expression of one or more gene encoding for H2A.Z, increasing the acetylation status of H2A.Z or preventing assembly of H2A.Z into the nucleosome as described herein.

As shown herein, inhibiting or decreasing the presence H2A.Z in the nucleosome in plants leads to an induction of responses that would normally be seen under high temperature conditions (about ≥22°C). The mutant plants phenocopy wild type plants growing at a higher temperature (around 5 °C higher than the actual temperature). Although a skilled person would appreciate that the temperature that determines a warm temperature response varies depending on the plant species, generally speaking, a warm temperature in plants response is a response that is shown at about 20 °C to about 24 °C, for example about ≥22°C. in Arabidopsis.

Thus, in one embodiment, crops that require a certain threshold temperature for proper growth and yield can be manipulated using the methods of the invention to reduce that temperature threshold. This is of particular benefit for crops that are grown in the greenhouse.

Another aspect of the disclosure relates to a method for inducing a cold temperature response in a eukaryotic organism comprising inhibiting or decreasing the eviction of H2A.Z from the nucleosome. The response may be constitutive. The method may include preventing acetylation status of H2A.Z or preventing disassembly of H2A.Z from the nucleosome as described herein. For example in plants, the mutant plants phenocopy wild type plants growing at a lower temperature (≤17 °C). Although a skilled person would appreciate that the temperature that determines a cold temperature response varies depending on the plant species, generally speaking, a cold temperature response in plants is therefore a response that is shown at about 15 °C to about 19 °C, for example about ≤17 °C in Arabidopsis. Other plants have different temperature optima as a skilled person will appreciate.

The disclosure also relates to methods for producing a eukaryotic organism, for example a plant, that shows a constitutive warm temperature response, even in cold temperatures and under conditions in which such response would not normally be displayed. Thus, in one embodiment, the invention relates to a method for producing a plant that shows a constitutive warm temperature response said method comprising inhibiting or decreasing the presence H2A.Z in the nucleosome. The method may include modifying the expression of one or more genes encoding for H2A.Z, increasing the acetylation status of H2A.Z or preventing assembly of H2A.Z into the nucleosome as described herein.

In another aspect, the disclosure relates to a method for producing a eukaryotic organism, for example in a plant, that shows a constitutive cold temperature response, even in high temperatures and under conditions in which such response would not normally be displayed. Thus, in one embodiment, the invention relates to a method for producing a plant that shows a constitutive cold temperature response said method comprising inhibiting or decreasing the eviction of H2A.Z from the nucleosome. The method may include preventing acetylation status of H2A.Z or preventing disassembly of H2A.Z from the nucleosome as described herein.

The invention also provides a plant H2A.Z molecule in which some or all of the acetylatable lysines of the molecule have been replaced by non-acetylatable amino acid residues.

The invention further provides a plant comprising a novel H2A.Z molecule in which some or all of the lysines of the molecule have been replaced by non-acetylatable amino acid residues or a nucleic acid sequence encoding such a molecule.

Furthermore, the invention provides a plant comprising a) a non-acetylatable version of histone H2A.Z;
b) an over-expressed histone deacetylase enzyme sufficient to remove histone acetylation marks in H2A.Z
or combinations thereof

According to the aspects above, the plant may be selected from the plants recited elsewhere herein.

Another object of this disclosure is to provide a method to enhance yield in crop plants by altering temperature sensing as according to the embodiments described above. In one embodiment, this aspect relates to a method to enhance grain-fill, grain composition or grain quantity in grain crops or to prevent premature flowering or bolting. In particular, the method relates to enhancing yield in crop plants by altering temperature sensing to allow said crop to reach its yield potential even at temperatures higher than those at which grain-fill is typically limited, or to avoid premature bolting/flowering following exposure to temperature conditions that typically cause such premature bolting.

As mentioned above, many harmful eukaryotic micro-organisms use temperature perception as a signal to induce pathogenesis, for example *Histoplasma capsulatum* and *Candida albicans* (Nguyen and Sil, 2008). Therefore, the ability to selectively perturb temperature perception in these organisms would provide means to prevent pathogenesis. Since the components of the deposition and eviction machinery are highly conserved, they make very good targets for small molecule inhibition. Furthermore, since the yeast would still be viable without undergoing pathogenesis, selection for resistant strains would be weak.

Therefore, in another aspect, the disclosure also relates to a method for inhibiting pathogenesis of a eukaryotic microorganism comprising altering temperature sensing in a eukaryotic organism comprising modifying the presence of H2A.Z in the nucleosome. Ways of altering temperature sensing are described herein. Moreover, small molecule therapies can be used to target the system.

Other objects and advantages of this invention will be appreciated from a review of the complete disclosure provided herein and the appended claims.

While the present invention has been generally described above, the following non limiting examples are provided to further describe the present invention, its best mode and to assist in enabling those skilled in the art to practice this invention to its full scope. The specifics of these examples should not be treated as limiting, however.

### EXAMPLES

### EXPERIMENTAL PROCEDURES

Unless otherwise stated herein, materials and methods used were as described here:

### Genetic screens for temperature perception

All experiments were carried out in Col-0 background. A forward genetic screen of fast neutron mutagenized population of *HSP70::LUC* reporter strain was carried out to identify mutants perturbed in the ambient temperature perception pathway (seeds irradiated at HAS KFKI-Atomic Energy Research Institute, Hungary). Seven-day-old seedlings were incubated at 12 °C for 48 h before shifting to 27 °C for three hours and screening for LUC expression by spraying with Luciferin. The *entr* mutations were identified as having significantly higher LUC induction than the reporter line. *ENTR1* was identified as *ARP6* through a microarray-based transcript analysis. Genomic fragment of containing *ARP6* genomic fragment (*P_{ARP6}::ARP6*) was used to complement *entr1* mutants.

### Transcript analysis

Transcript analyses were done on total RNA was extracted using Trizol (Invitrogen) Labeled targets were hybridized to Affymetrix ATH1 array according to the manufacturer's instructions. Microarray data were analyzed using GenespringGX 7.3 (Agilent).

### Chromatin Immuno-purification (ChIP)

ChIP was performed as described (Gendrel et al., 2002) with minor modifications. Seven-day-old seedlings grown at 17°C were shifted to 27°C for 2 hours and ChIP experiments were performed in parallel on chromatin from both temperatures. For H2A.Z and H3 ChIP, cross-linked chromatin was fragmented with 0.2 units of Micrococcal nuclease (Sigma) in 1 ml of MNase digestion buffer (10 mM Tris-HCl [pH 8.0], 50 mM NaCl, 1 mM β-mercaptoethanol, 0.1% NP40, 1 mM CaCl₂ and 1x protease inhibitor cocktail [Roche]). Digestion was stopped using 5 mM EDTA. HTA11, one of the Arabidopsis H2A.Z homologues, was GFP tagged and ChIP was performed using GFP polyclonal antibody (Abcam, ab290). Histone H3 dynamics were assayed using H3 antibody (Abcam, ab1791). For RNA polymerase II occupancy dynamic experiments chromatin was fragmented by sonication in lysis buffer (10 mM Tris-HCl [pH 8], 150 mM NaCl, 1 mM EDTA [pH 8], 0.1% deoxycholate and 1x protease inhibitor cocktail) and ChIP was performed using monoclonal antibody to RNA polymerase II CTD repeat YSPTSPS (Abcam, ab817). All ChIP experiments were performed in buffer containing 10 mM Tris-HCl [pH 8.0], 5 mM EDTA [pH 8.0], 150 mM NaCl, 1 % Triton X-100 and 1x protease inhibitor cocktail. Relative enrichment of associated DNA fragments were analysed by quantitative PCR (qPCR). All oligonucleotide sequences used for target DNA detection and quantification in ChIP experiments are given in supplementary material. Each ChIP experiment was repeated at least three times and the data presented are from a representative experiment.

### Nucleosome positioning

Micrococcal nuclease (MNase) digestion of enriched chromatin followed by qPCR using tiled oligonucleotides surrounding the transcription start site (TSS) was used for nucleosome positioning and analysis. Oligos were designed to have 95-110 bp amplicons every 35-45 bp in the HSP70 promoter. For nucleosome positioning, chromatin from seven-day-old seedlings of Col-0 and *entr1* grown at varying temperatures (17 °C, 22 °C, 27 °C) were digested with MNase and mononucleosome sized fragments were gel purified and used in qPCR. Relative nucleosome occupancy was represented as fraction of uncut chromatin DNA and was plotted against the *HSP70* gene position with respect to the TSS for each primer pair where the position denotes the center of each amplicon.

### Nucleosome purification by hydroxyapatite chromatography

Seedlings growing at 17 °C were frozen in liquid nitrogen without cross-linking and chromatin was prepared as for the ChIP experiments. Following MNase digest for 15 min at 17 °C, chromatin fragments were bound to hydroxyapatite and nucleosomes devoid of linker histones and other associated proteins were purified according to (Brand et al., 2008). Purified nucleosomes were buffer exchanged to 10 mM Tris-HCl (pH 8.0), 1 mm EDTA (pH 8.0), 25 mM NaCl and 1x protease inhibitor cocktail. Approximately 500 ng equivalent of nucleosomal DNA was assayed for restriction enzyme accessibility at 17 °C and 27 °C. The fraction of the input nucleosomal DNA protected was obtained using qPCR for HSP70 -1 and +1 nucleosomes. Data represented are normalized against the +1 nucleosome of At4g07700 that does not have a restriction site.

### EXAMPLE 1

### HSP70 is an output of the ambient temperature sensing pathway

To identify the major ambient temperature responses in seedlings, we analysed the transcriptomes of 12 day old plants grown at 12 °C and shifted to 27 °C. We found that 2454 genes are upregulated at least 2-fold under these conditions and 2880 genes are 2-fold down-regulated (the ambient temperature transcriptome; Figure 1A). As in similar studies, there was not a significant induction of stress markers, suggesting that this temperature range is not causing heat stress (Balasubramanian et al., 2006). We found that *HSP70* (At3g12580), is strongly up-regulated at higher temperature (Figure 1B). While this transcriptome is characterised by a response to ambient temperature change, we wanted to determine if any of these genes were also responsive to differences in constant growth temperature, since the transcriptional output of a thermosensory pathway should be different at various constant temperatures. We therefore analysed the behaviour of the induced ambient temperature transcriptome genes in publicly available data for plants grown at 12 °C, 17 °C, 22 °C and 27 °C ( NASCARRAYS-147 (http://affymetrix.arabidopsis.info/narrays/experimentpage.pl? experimentid=147).). We found *HSP70* is expressed at a level proportionate to the ambient temperature within this temperature range (Figure 1C). While these experiments were performed on seedlings, *HSP70* also shows these expression dynamics in the adult plant (Balasubramanian et al., 2006). We therefore used a fusion of the *HSP70* promoter to Luciferase *(HSP70::LUC)* to monitor the activity of the endogenous gene non-destructively. *HSP70::LUC* recapitulates the expression of *HSP70* (Figure 1D), providing us with a dynamic and sensitive assay for temperature perception status *in planta,* independent of high temperature stress (Larkindale and Vierling, 2008; Sung et al., 2001).

### EXAMPLE 2

### ARP6 is in the ambient temperature sensing pathway that controls flowering

To identify genes required to control the ambient temperature transcriptome, we screened 2,600 individual M2 families of fast neutron irradiated *HSP70::LUC* seedlings. Two mutations, *entr1* and *entr2 (enhanced temperature response1 and 2),* displayed a constitutively higher *LUC* expression (Figure 1E). Genetic analysis using a complementation cross revealed that these mutations are allelic. Transcript based cloning revealed that the *ARP6* transcript is absent in both *entr1* and *entr2* (Figure 1F). Transformation of *entr1* with a genomic fragment of *ARP6* is able to rescue both the *HSP70::LUC* expression level as well as the altered development of *entr1* (Figure 1G), confirming that it is a new *arp6* allele. We will refer to *entr1* and *entr2* as *arp6*-*10* and *arp6-11* respectively. Arabidopsis mutants in *ARP6* have been identified in flowering time screens (Choi et al., 2005; Deal et al., 2005; Martin-Trillo et al., 2006). To determine if *ARP6* acts in the ambient temperature pathway for flowering, we analysed flowering in *arp6-10.* Consistent with earlier reports, *arp6*-*10* flowers earlier at 21 °C in long and 22 °C in short days (Figure 2A-2D). We then studied the response of *arp6*-*10* to 27 °C in short days, since the thermal induction of flowering is most pronounced in short days (Balasubramanian et al., 2006). Remarkably, *arp6*-*10* flowers with about 5 leaves in short days at 27 °C (Figure 2E and 2F). *ARP6* therefore acts in the ambient temperature pathway that controls flowering.

### EXAMPLE 3

### ARP6 controls developmental responses to ambient temperature globally

Specific adaptive changes occur to plant architecture in response to higher ambient temperature, including increases in hypocotyl growth and petiole elongation (Gray et al., 1998; Koini et al., 2009). We analysed these traits to see if *arp6-*10 exhibits a global high temperature response in its architecture as well as *HSP70* expression and flowering time. We find that architecture responses are strongly enhanced in the *arp6* background (Figure 2G and 2H and FIGURE 8), such that *arp6* plants grown at 17 °C exhibit greater hypocotyl elongation and petiole growth than wild-type plants grown at 22 °C, with an equivalent difference between 22 °C and 27 °C. These phenotypes have been shown to be dependent on the PIF4 transcription factor (Koini et al., 2009), so it is not surprising that we still observe a temperature-induced difference, even in the *arp6*-*10* mutant. A functional *ARP6* is required however for controlling the correct level of expression of these phenotypes. A longstanding observation in plant biology is that thermal time is a key measure for the rate of transition through the developmental phases, and that phase transition is accelerated by higher ambient temperature (Poethig, 2003). In addition to the flowering time acceleration in *arp6*-*10*, we observe a more rapid juvenile to adult transition (data not shown), consistent with earlier studies (Martin-Trillo et al., 2006). Thus *arp6*-*10* causes a specific up-regulation of all the developmental decisions known to be regulated by temperature that we have examined. Plants deficient in *ARP6* therefore display a constitutive warm temperature developmental program.

### EXAMPLE 4

### The ambient temperature transcriptome is globally misregulated in arp6

To determine if the global mis-regulation of developmental responses in the absence of *ARP6* occurs at the transcriptional level, we compared the ambient temperature transcriptomes of *arp6*-*10* and wild-type. While 2,454 genes are upregulated 2-fold or more on shifting from 12 °C to 27 °C in wild-type (Figure 3A, grey bars), we observe this ambient temperature transcriptome is consistently expressed in *arp6*-*10*, even at 12 °C (Figure 3A, blue bars). Interestingly, this effect reflects more than a constitutive up-regulation of gene expression *per se,* since the 2880 genes that are down regulated at least 2-fold on increasing temperature in wild-type (Figure 3B, grey bars) are also constitutively repressed in *arp6*-*10*. Thus, transcriptionally, the *arp6*-*10* mutant at 12 °C resembles a plant at a higher temperature. In a reciprocal analysis, transcripts that are 2-fold more highly expressed at 12 °C in *arp6*-*10* compared to wild-type (Figure 3C, blue bars) are strongly induced by higher ambient temperature in wild-type, while those genes that are repressed in *arp6-10* compared to wild-type at 12 °C (Figure 3D, blue bars), are transcriptionally repressed by higher temperature in wild-type (Figure 3D, grey bars). These results show that in the absence of *ARP6,* the ambient temperature regulated genes are constitutively mis-expressed. To determine what proportion of the entire ambient temperature transcriptome is *ARP6* dependent we compared the change of gene expression in response to higher ambient temperature in wild-type with the change in expression resulting from *arp6*-*10* at constant temperature. Remarkably, there is a strong correlation for the entire transcriptome: a functional *ARP6* allele accounts for almost half of the ambient temperature transcript responses (R² = 0.466, P < 0.001). *ARP6* is therefore necessary for coordinating the transcriptome in response to ambient temperature.

### EXAMPLE 5

### H2A.Z occupancy changes with temperature

*ARP6* encodes a subunit of the SWR1 complex that is conserved among eukaryotes and is necessary for inserting the alternative histone H2A.Z into nucleosomes in place of H2A (Deal et al., 2007; Kobor et al., 2004; Krogan et al., 2003; Mizuguchi et al., 2004). There are several H2A.Z family members in Arabidopsis, and we find the *hta9 hta11* double mutant phenocopies *arp6*-*10*, showing the early flowering and architectural responses as well as increased *HSP70* expression. This indicates that the temperature responses of *arp6*-*10* result from a failure to incorporate H2A.Z-containing nucleosomes into the genome. Global analyses in yeast, Drosophila, *C. elegans,* Arabidopsis and mammalian cells have revealed that H2A.Z-containing nucleosomes are especially enriched at the +1 position near the transcriptional start site (Creyghton et al., 2008; Mavrich et al., 2008; Raisner et al., 2005; Whittle et al., 2008; Zhang et al., 2005; Zilberman et al., 2008). H2A.Z has been implicated in maintaining promoters of quiescent genes in a poised state ready for appropriate transcription (Li et al., 2005). A possible explanation for the central role of *ARP6* in regulation of the temperature transcriptome is that H2A.Z-containing nucleosomes confer a temperature dependence on transcription. To test if H2A.Z dynamics change in response to temperature, we used chromatin immunopurification (ChIP) to follow H2A.Z occupancy at the *HSP70* promoter in response to different ambient temperatures. We used *HTA11:GFP* expressed under the endogenous *HTA11* promoter for our ChIP experiments. This construct complements the *hta9 hta11* double mutant (Figure 4A), showing that *HTA11:GFP* is functional. Microscopy of HTA11:GFP reveals incorporation into euchromatin as in other studies (Figure 9A and 9B) (Zilberman et al., 2008).

ChIP analysis revealed a greatly depleted H2A.Z occupancy at the *HSP70* promoter in *arp6*-*10* (Figure 9C), suggesting that the increased *HSP70* expression in *arp6-10* is a consequence of this change in chromatin architecture, as indicated by the *hta9 hta11* phenotype. To see if temperature affects H2A.Z occupancy at *HSP70,* we compared seedlings grown at 17 °C with seedlings shifted to 27 °C for two hours. Consistently, at 17 °C, where expression of *HSP70* is low, we observe a high occupancy of H2A.Z at the +1 nucleosome (Figure 4B). This occupancy is however very temperature dependent, with H2A.Z occupancy decreasing sharply at 27 °C compared to 17 °C (Figure 4B). We have validated our ChIP results using an H2A.Z antibody (Deal et al., 2007) that gives similar results (Figure 9D). These results are consistent with a model where H2A.Z occupancy is rate limiting for the transcription of temperature responsive genes, and the occupancy of H2A.Z responds to temperature. To determine whether the behaviour we observe is specific to H2A.Z or is a general property of nucleosomes in response to temperature, we carried out parallel ChIP studies using histone H3 specific antibody. Upon shifting to 27 °C, H3 levels at the +1 nucleosome drop, though not to the extent of H2A.Z (Figure 4C). Interestingly at the -1 nucleosome, where H2A.Z occupancy is reduced, H3 levels did not drop significantly. This suggests that H2A.Z-containing nucleosomes exhibit specific dynamic responses to temperature. Since RNA Pol II must negotiate +1 nucleosomes for transcription to occur, this suggests a model where gene transcription may be controlled by the temperature responsive occupancy of +1 nucleosomes.

The changes we observe in H3 occupancy at *HSP70* may be a consequence of H2A.Z nucleosomes having a temperature specific response. We therefore extended our analysis to the TSS of another locus, At4g07700, chosen since it does not contain H2A.Z (Zilberman et al., 2008) and it is largely transcriptionally silent, avoiding complicating factors arising from RNA Pol II mediated displacement of nucleosomes, as well as being predicted to have a well ordered nucleosome structure using a recent model (Kaplan et al., 2009). ChIP analysis with H3 specific antibody showed the predicted nucleosome occupancy. ChIP analysis revealed there was no significant change in H3 occupancy at this locus upon shifting to 27 °C (Figure 4D). This is consistent with our findings for the -1 nucleosome of *HSP70* where there is little or no H2A.Z incorporation, and H3 levels do not show significant temperature responsive dynamics. These results suggest that H2A.Z is the major determinant temperature responsiveness of chromatin.

To test if this response is unique to *HSP70,* we examined the TSS proximal regions corresponding to the presumptive +1 nucleosomes of a number of other temperature up-regulated genes (Figure 5A-5C). Consistently, all these genes show a sharp reduction in H2A.Z occupancy at 27 °C. Since it has been shown that *FT* is an essential output of the thermosensory pathway for mediating early flowering, we sought to see if H2A.Z is present in the *FT* promoter and if it is thermoresponsive. Although *FT* is not significantly expressed in seedlings, eviction of H2A.Z may be necessary to confer transcriptional competence on the *FT* locus. Analysis of global H2A.Z occupancy data (Zilberman et al., 2008) revealed that H2A.Z is enriched in the *FT* promoter. We therefore assayed for H2A.Z changes at *FT* in response to temperature change. Consistently, we observe H2A.Z depletion from the *FT* promoter at higher temperatures (Figure 9E), suggesting an explanation for the acceleration of flowering in *arp6.*

### EXAMPLE 6

### Eviction of H2A.Z occurs independently of transcription

We have shown that H2A.Z occupancy decreases dynamically with increasing temperature at the +1 nucleosomes of temperature-induced genes. Since temperature-dependent histone eviction may be a consequence of greater transcriptional activity, and is not necessarily the cause, we sought to determine if H2A.Z eviction occurs independently of transcription. To test this, we analysed genes that are enriched for H2A.Z in their promoters and are either down regulated or show constant expression in response to increased temperature. We then performed ChIP to assay H2A.Z occupancy in response to ambient temperature change (Figure 5D-5I). We observed a significant decrease in H2A.Z occupancy across all the genes examined, independent of their transcriptional response to temperature. This result suggests H2A.Z-containing nucleosomes have a binding affinity or occupancy as measured by ChIP that varies with temperature. This is independent of transcription, which is analogous to the behaviour of nucleosomes at the Hsp70 locus of Drosophila (Petesch and Lis, 2008). Since the presence of H2A.Z-containing nucleosomes is rate-limiting for the expression of the majority of the genes in the ambient temperature transcriptome, this suggests that H2A.Z responses to temperature play a key role in integrating temperature information.

### EXAMPLE 7

### The HSP70 +1 nucleosome shows a constitutive temperature response in arp6

Since we have found that H2A.Z nucleosomes are evicted at higher temperature, we analysed the nucleosome structure of the *HSP70* promoter at different temperatures by studying MNase accessibility. MNase digests nucleosome-free DNA and the linker regions between nucleosomes, while the DNA of the nucleosomes themselves is protected from digestion (Petesch and Lis, 2008). For plants grown at 17 °C, 22 °C and 27 °C, we observed a sharp drop in nucleosome occupancy with greater temperature (Figure 6A), which helps us understand how *HSP70* transcription increases with temperature. By comparison, the +1 nucleosome of *HSP70* in the *arp6* background is constitutively in the open conformation, even at 17 °C (Figure 6B), at which temperature there is significant nucleosome occupancy in wild-type. At higher temperatures, we also observe a decrease in the -1 occupancy, which is likely a consequence of greater transcriptional activity. This decrease in -1 occupancy appears to be H2A.Z independent. Consistently, the nucleosome structure of At4g07700, which does not contain H2A.Z nucleosomes, does not change with temperature (Figure 6C).

The results so far are consistent with a model where expression of *HSP70* may be regulated at the level of nucleosome occupancy. For RNA Pol II to transcribe target genes, it must be able to overcome the physical barrier posed by nucleosomes (Knezetic and Luse, 1986; Lorch et al., 1987).

Numerous studies indicate that nucleosomes at genes induced to undergo transcription dissociate or partially unwrap from their DNA, allowing passage of RNA Pol II, and the presence of nucleosomes, especially the +1 nucleosome near the transcriptional start site, can be rate limiting for controlling transcription (Boeger et al., 2008; Boeger et al., 2003). Under heat shock conditions, nucleosomes at the Drosophila Hsp70 locus are lost from the body of the gene in advance of the arrival of RNA Pol II, indicating that the nucleosomes are able to play a role in regulating transcription (Petesch and Lis, 2008).

RNA Pol II ChIP was used to study transcriptional dynamics at *HSP70* in wild-type and *arp6*-*10*. At 17 °C in wild-type, we observed a greater proportion of RNA Pol II present at the TSS compared to in the body of the gene. By comparison, in *arp6* a greater proportion of RNA Pol II is present in the body of the gene (Figure 6D). At 27 °C there is a strong shift of RNA Pol II occupancy, with the highest peak now occurring downstream of the TSS. Since this new RNA Pol II peak corresponds to the region occupied by the +1 nucleosome, it is likely that in wild-type at non-inductive conditions the +1 nucleosome plays a role in maintaining transcription in a poised state. Upon increasing temperature, the presence of Pol II on the gene body increases considerably (Figure 6E). This is consistent with studies in *C*. *elegans* and *Drosophila,* which have suggested that H2A.Z +1 nucleosomes may maintain RNA Pol II in a poised state (Mavrich et al., 2008; Whittle et al., 2008). The presence of H2A.Z containing nucleosomes is therefore rate-limiting for the upregulation of *HSP70.*

### EXAMPLE 8

### H2A.Z nucleosomes wrap DNA more tightly

We have found that in the absence of H2A.Z incorporation, canonical H2A containing nucleosomes are unable to prevent constitutive expression of the warm temperature transcriptome of both up- and down-regulated genes. This difference could result directly from a difference in the behaviour of H2A.Z nucleosomes, or may reflect the effect of other factors recruited by H2A.Z. To distinguish between these two possibilities, we purified nucleosomes from plants grown at 17 °C using hydroxyapatite chromatography following a partial MNase digest. It has recently been demonstrated that RNA Pol II does not actively unwrap nucleosomes, but depends on fluctuations that locally unwrap DNA for it to transcribe its template (Hodges et al., 2009). Since the invasion of RNA Pol II into nucleosomal DNA is the rate limiting step for transcription, we assayed the exposure of restriction enzyme sites occluded by H2A.Z and H2A nucleosomes at 17 °C and 27 °C (Figure 6F and 6G). This assay provides a direct measure of the degree of local unwrapping and exposure of nucleosomal DNA (Polach and Widom, 1995). The H2A-containing -1 nucleosome of *HSP70* displays a greater accessibility as compared to the H2A.Z containing +1 nucleosome at both 17 °C and 27 °C. This greater accessibility of H2A-containing nucleosomes provides an explanation for the greater transcription of *HSP70* in the *arp6* background where H2A.Z is not incorporated into chromatin. The rate of exposure of these sites appears to increase with higher temperature, suggesting that local fluctuations of the nucleosomal DNA increase with temperature. Since there might be subtle differences in the accessibility of the *Hph* I site between the -1 and +1 nucleosomes, we purified nucleosomes from *arp 6.* In this material both nucleosomes contain H2A, and as we would predict, they are both equally accessible (Figure 6H). These results show that H2A.Z containing nucleosomes wrap DNA more tightly, and higher temperatures may overcome this. Since these nucleosomes were purified from plant material, we do not exclude that post-translational modifications, for example acetylation, may be involved in modulating this response. Importantly, our assay is performed on highly purified material, indicating that the dynamic responses we observe are an inherent property resulting from the nucleosome-DNA interactions. Our finding that the degree of local unwrapping of DNA on H2A.Z containing nucleosomes is reduced compared to H2A nucleosomes suggests a direct mechanism by which transcription can be adjusted according to the temperature of the cell.

### EXAMPLE 9

### The role of H2A.Z in controlling the temperature transcriptome is conserved

Since temperature changes represent a fundamental challenge to all sessile organisms, we sought to determine if control of temperature transcriptomes might be a conserved function of the H2A.Z nucleosomes in eukaryotes generally. To test this, we analysed the role of the budding yeast H2A.Z, Htz1, in temperature signalling. The Htz1 transcriptome has been previously determined (Meneghini et al., 2003), and we therefore tested to see if those genes that are mis-regulated in the *htz1Δ* background also show a corresponding change when wild-type yeast are shifted from 29 °C to 33 °C (Gasch et al., 2000). Strikingly, we see a significant correlation (R² = 0.30, P < 0.001), with genes that are up-regulated at higher temperatures tending to also be upregulated in *htz1Δ* and *vice versa* (Figure 10). This is the same relationship we have observed in Arabidopsis (Figure 3E), showing that the loss of H2A.Z containing nucleosomes phenocopies cells at higher temperature. These data suggest that warm temperature signals are mediated through H2A.Z nucleosomes in yeast as well as plants.

### EXAMPLE 10

### Expression non-acetylatable (mutant) version of H2A.Z

Arabidopsis encodes three major H2A.Z genes (*HTA8, HTA9* and *HTA11*)*.* Mutation of the two major genes *HTA9* and *HTA11* (in *hta9 hta11* double mutants) phenocopy arp6 mutant indicative of enhanced temperature reponse. We have been able to complement the *hta9 hta11* double mutant phenotype by expressing HTA11 from its native promoter.

A 2786 bp genomic region of *HTA11* (At3g54560) including 1589 bp promoter region and coding sequences (genomic cordinates Chromosome 3: 20194677 - 20197463) was amplified using oligos W1160 (5'CACCGAAATGTTTTTCTCTACG 3') and W1161 (5'CTCCTTGGTGGTTTTGTTGA 3'). The amplified fragment was cloned into pENTR vector (invitrogen) according to the manufacturer's instructions. pENTR-*HTA11* was then used for further mutagenesis experiments. PCR mediated site-directed mutagenesis was used to mutate conserved lysine residues in the N-terminal tail of *HTA11.*

Lysine residues at positions 4,7, 13, 19 and 21 of HTA11 N-teminus were selected for mutagenesis. The wild type HTA11 with all five lysine residues intact is hereafter mentioned HTA11-KKKKK.

Oligos W1443(5' TCAAGAGACATGGCAGGCAGAGGTGGAAGAGGACTCGTAGCTGCG 3') and W1444 (5' CGCAGCTACGAGTCCTCTTCCACCTCTGCCTGCCATGTCTCTTGA 3') were used for targeted mutagenesis of lysine's residues 4 (K₄) and K₇ to arginines (R) using HTA11-KKKKK as template. The resulting plasmid was named HTA11-RRKKK.

Oligos W1445(5' GGACTCGTAGCTGCGAGGACGATGGCTGCTAAC 3') and W1446 (5' GTTAGCAGCCATCGTCCTCGCAGCTACGAGTCC 3') were used for targeted mutagenesis of lysine's residues 13 (K₁₃) to arginines (R)using HTA11-KKKKK as template. The resulting plasmid was named HTA11-KKRKK.

Oligos W1447(5' GACGATGGCTGCTAACAGGGACAGAGACAAGGACAAGAAG 3') and W1448 (5' CTTCTTGTCCTTGTCTCTGTCCCTGTTAGCAGCCATCGTC 3') were used for targeted mutagenesis of lysine's residues 19 and 21 (K₁₉, K₂₁) to arginines (R)using HTA11-KKKKK as template. The resulting plasmid was named HTA11-KKKRR.

Oligos W1445(5' GGACTCGTAGCTGCGAGGACGATGGCTGCTAAC 3') and W1446 (5' GTTAGCAGCCATCGTCCTCGCAGCTACGAGTCC 3') were used for targeted mutagenesis of lysine's residues 13 (K₁₃) to arginines (R)using HTA11-RRKKK as template. The resulting plasmid was named HTA11-RRRKK.

Oligos W1447(5' GACGATGGCTGCTAACAGGGACAGAGACAAGGACAAGAAG 3') and W1448 (5' CTTCTTGTCCTTGTCTCTGTCCCTGTTAGCAGCCATCGTC 3') were used for targeted mutagenesis of lysine's residues 19 and 21 (K₁₉, K₂₁) to arginines (R) using HTA11-RRRKK as template. The resulting plasmid was named HTA11-RRRRR.

Wild type (HTA11-KKKKK) and mutagenized versions of HTA11 (HTA11-RRKKK, HTA11-KKRKK, HTA11-KKKRR, HTA11-RRRKK and HTA11-RRRRR) were transferred to a binary gateway vector pW1357 by recombination so as to have c-terminus fusions of the proteins with 3xFLAG tag. FLAG tagged wild type and mutant versions of HTA11 are used to transform hta9 hta11 double mutant as well as Col-0 wild type.

### EXAMPLE 11

### Knocking out (or knocking down) acetyltransferase enzyme gene

The HAC family of Arabidopsis histone acetyl transferase family consists of five genes At1g79000 *HACl,* At1g67220 (*HAC2),* At1g55970 (*HAC4*), At3g12980 (*HAC5*) and At1g16710 (*HAC12*). Artificial microRMA (miRNA) design using Web MicroRNA Designer (http://wmd3.weigelworld.org/cgi-bin/webapp.cgi). miRNA was amplified as suggested
(http://wmd3.weigelworld.org/downloads/Cloning_of_artificial_m icroRNAs.pdf) using oligos . Amplified miRNA was cloned into pENTR vector and there after into binary transformation vector pW545 through recombination to create 35S::HACmiRNA(pW1009). Agrobacterium strains harboring the binary vector pW1009 was used to transform Arabidopsis Col-0. Preliminary analysis of the transgenic lines revealed a delayed flowering phenotype consistent with the expectation. These transgenic lines are analysed in detail to characterize ambient temperature response.

### EXAMPLE 12

### Overexpressing deacetylase enzymes

Protein coding sequences of one or more of the histone deacetylase (HDAC) genes are amplified from a cDNA library, and are cloned into pENTR vector and there after transferred to a binary vector under the control of a constitutive CaMV35S promoter. Arabidopsis plants were transformed with the binary vector harbouring Agrobacterium to obtain transgenic lines.

### Nucleosomes as temperature responsive regulators of transcription

Nucleosomes play an active role in controlling transcriptional processes through modulating the ability of transcription factors to access their cis elements or occluding the passage of RNA Pol II (Lam et al., 2008; Segal and Widom, 2009). Access of proteins to DNA wrapped around histones is mediated through local unwrapping events. This unwrapping is not affected by changes in ambient temperature in our study and others for H2A-containing nucleosomes (Polach and Widom, 1995). By contrast, H2A.Z-containing nucleosomes exhibit a much tighter wrapping of their DNA, consistent with studies of nucleosomal stability (Thambirajah et al., 2006). This is supported by the observation that H2A.Z-containing nucleosomes are more tightly positioned in the genome and are less fuzzy. Work on reconstituted nucleosomal arrays has also shown that the intranucleosomal interactions are stronger in H2A.Z-containing nucleosomes, while these have weaker internucleosomal interactions (Fan et al., 2002). In addition to H2A.Z-containing nucleosomes having more tightly wrapped DNA, our results suggest that the degree of unwrapping may also be responsive to temperature. This result suggests a direct mechanism by which temperature may influence gene expression, since it has been shown that RNA Pol II does not actively invade nucleosomes, but waits for local unwrapping of DNA from nucleosomes before extending transcription (Hodges et al., 2009). In this way, genes with a paused RNA Pol II will show increased transcription with greater temperature as local unwrapping is increased (Figure 7). For those genes whose transcription decreases with temperature, we propose greater temperature increases the frequency with which transcriptional repressors may bind. The presence of H2A.Z-containing nucleosomes in these genes may prevent binding of a transcriptional repressor, or may antagonise DNA methylation which has been shown to be a role of H2A.Z in plants (Zilberman et al., 2008). Consistently, H2A.Z-containing nucleosomes have been shown to play an important role in anti-silencing (Meneghini et al., 2003).

A large number of genes that are both up- and down-regulated in *arp6*-*10* and exhibit a corresponding change with increased temperature. Furthermore, we observe the same de-regulation of the warm temperature transcriptome in the *htz1Δ* mutant in yeast, suggesting this is a conserved function of H2A.Z containing nucleosomes. Importantly, there are many physiological responses to temperature, and undoubtedly many mechanisms for sensing and responding to temperature in different organisms. For example the PIF4 transcription factor mediates the high temperature architecture phenotypes in plants (Koini et al., 2009). Our mechanism for H2A.Z-containing nucleosomes provides an attractive model for how these transcriptional responses may be integrated with temperature status. On theoretical grounds it has been shown that regulating transcription at multiple levels greatly improves robustness and adaptivity (Tsankov et al., 2006).

### Connections between thermosensing and flowering pathways

Arabidopsis thaliana has a highly plastic life history, and a genetically informed photothermal model is able to explain most of the variation in flowering time for plants grown at different seasons (Wilczek et al., 2009). Acceleration of flowering in response to higher temperature requires genes in the autonomous pathway (Blazquez et al., 2003), and is dependent on increasing expression of FLOWERING LOCUS T (FT) (Balasubramanian et al., 2006; Cerdan and Chory, 2003; Halliday et al., 2003; Lee et al., 2007).

Flowering is one of the major developmental pathways regulated by ambient temperature. It has been shown that two autonomous flowering pathway genes, *FVE* and *FCA,* are necessary for mediating the ambient temperature response for flowering (Blazquez et al., 2003). Interestingly, an *arp6* allele, *esd1-*2, is epistatic to *fve* and *fca* (Blazquez et al., 2003; Martin-Trillo et al., 2006). *FVE* is a homolog of the mammalian retinoblastoma-associated protein, a component of a histone deacetylase complex (Ausin et al., 2004; Kim et al., 2004). As well as affecting the autonomous pathway, which regulates flowering time through the floral pathway integrator *FLOWERING LOCUS C (FLC), FVE* is also involved in sensing cold temperatures (Kim et al., 2004). Taken together, these data suggest that *FVE* may exert its effects on temperature-dependent pathways through modulating H2A.Z, perhaps through acetylation, which affects nucleosome stability (Ishibashi et al., 2009; Thambirajah et al., 2006). Although the thermosensory flowering pathway is mediated by *FT* expression levels, the major regulator of *FT* expression in response to long photoperiods, *CONSTANS (CO),* is not essential for perceiving temperature, since *co-1* responds to thermal induction of flowering (Balasubramanian et al., 2006). Our observation that the chromatin status at the *FT* locus responds to temperature and is altered in the absence of H2A.Z provides an explanation for how the thermal induction pathway may activate *FT* expression in response to higher temperature independently of CO. Interestingly, another temperature dependent pathway that also affects flowering time in plants, vernalisation, is also perturbed in the *arp6* background (Choi et al., 2005; Deal et al., 2007). A vernalisation requirement in certain Arabidopsis genotypes is suppressed by *arp 6.* This effect is mediated through *FLC,* a floral repressor whose expression is dependent on the SWR1 complex. Interestingly, in a large field experiment it was found that natural temperature fluctuations were likely to be sufficient for overcoming *FLC* induced repression of flowering in Arabidopsis (Wilczek et al., 2009). Temperature fluctuations of this type are likely to signal through H2A.Z dynamics, which is consistent with the observation that perturbation of H2A.Z in *arp6* is sufficient to overcome a vernalisation requirement (Choi et al., 2005; Deal et al., 2005; Martin-Trillo et al., 2006).

### Temperature perception and climate change

While many plants have shown a rapid acceleration of flowering time in response to climate change (Fitter and Fitter, 2002), species that do not are going locally extinct at a high rate (Willis et al., 2008). This indicates that phenological responses to temperature change are of high adaptive value. A molecular understanding of the mechanism of temperature perception will enable us to understand how different species will respond to further increases in temperature and be a key step towards breeding crops able to withstand climate change.

### Example 13

### Transformations of barley and brassica

Barley plants are transformed with the vector pBRACT214 for constitutive overexpression of the altered H2A.Z non-acetylatable versions under the Ubiquitiin promoter. Brassica are transformed with the vector pBRACT103 for expression of non-acetylatable H2A.Z versions of H2A.Z.

### REFERENCES:

Ausin, I., Alonso-Blanco, C., Jarillo, J.A., Ruiz-Garcia, L., and Martinez-Zapater, J.M. (2004). Regulation of flowering time by FVE, a retinoblastoma-associated protein. Nat Genet 36, 162-166.
Balasubramanian, S., Sureshkumar, S., Lempe, J., and Weigel, D. (2006). Potent induction of Arabidopsis thaliana flowering by elevated growth temperature. PLoS Genet 2, e106.
Blazquez, M.A., Ahn, J.H., and Weigel, D. (2003). A thermosensory pathway controlling flowering time in Arabidopsis thaliana. Nat Genet 33, 168-171.
Boeger, H., Griesenbeck, J., and Kornberg, R.D. (2008). Nucleosome retention and the stochastic nature of promoter chromatin remodeling for transcription. Cell 133, 716-726.
Boeger, H., Griesenbeck, J., Strattan, J.S., and Kornberg, R.D. (2003). Nucleosomes unfold completely at a transcriptionally active promoter. Mol Cell 11, 1587-1598.
Brand, M., Rampalli, S., Chaturvedi, C.P., and Dilworth, F.J. (2008). Analysis of epigenetic modifications of chromatin at specific gene loci by native chromatin immunoprecipitation of nucleosomes isolated using hydroxyapatite chromatography. Nat Protoc 3, 398-409.
Cerdan, P.D., and Chory, J. (2003). Regulation of flowering time by light quality. Nature 423, 881-885.
Choi, K., Kim, S., Kim, S.Y., Kim, M., Hyun, Y., Lee, H., Choe, S., Kim, S.G., Michaels, S., and Lee, I. (2005). SUPPRESSOR OF FRIGIDA3 encodes a nuclear ACTIN-RELATED PROTEIN6 required for floral repression in Arabidopsis. Plant Cell 17, 2647-2660.
Creyghton, M.P., Markoulaki, S., Levine, S.S., Hanna, J., Lodato, M.A., Sha, K., Young, R.A., Jaenisch, R., and Boyer, L.A. (2008). H2AZ is enriched at polycomb complex target genes in ES cells and is necessary for lineage commitment. Cell 135, 649-661.
Deal, R.B., Kandasamy, M.K., McKinney, E.C., and Meagher, R.B. (2005). The nuclear actin-related protein ARP6 is a pleiotropic developmental regulator required for the maintenance of FLOWERING LOCUS C expression and repression of flowering in Arabidopsis. Plant Cell 17, 2633-2646.
Deal, R.B., Topp, C.N., McKinney, E.C., and Meagher, R.B. (2007). Repression of flowering in Arabidopsis requires activation of FLOWERING LOCUS C expression by the histone variant H2A.Z. Plant Cell 19, 74-83.
Fan, J.Y., Gordon, F., Luger, K., Hansen, J.C., and Tremethick, D.J. (2002). The essential histone variant H2A.Z regulates the equilibrium between different chromatin conformational states. Nat Struct Biol 9, 172-176.
Fitter, A.H., and Fitter, R.S. (2002). Rapid changes in flowering time in British plants. Science 296, 1689-1691. Gasch, A.P., Spellman, P.T., Kao, C.M., Carmel-Harel, O., Eisen, M.B., Storz, G., Botstein, D., and Brown, P.O. (2000). Genomic expression programs in the response of yeast cells to environmental changes. Mol Biol Cell 11, 4241-4257.
Gendrel, A.V., Lippman, Z., Yordan, C., Colot, V., and Martienssen, R.A. (2002). Dependence of heterochromatic histone H3 methylation patterns on the Arabidopsis gene DDM1. Science 297, 1871-1873.
Gray, W.M., Ostin, A., Sandberg, G., Romano, C.P., and Estelle, M. (1998). High temperature promotes auxin-mediated hypocotyl elongation in Arabidopsis. Proc Natl Acad Sci U S A 95, 7197-7202.
Halliday, K.J., Salter, M.G., Thingnaes, E., and Whitelam, G.C. (2003). Phytochrome control of flowering is temperature sensitive and correlates with expression of the floral integrator FT. Plant J 33, 875-885.
Heggie, L., and Halliday, K.J. (2005). The highs and lows of plant life: temperature and light interactions in development. Int J Dev Biol 49, 675-687.
Hodges, C., Bintu, L., Lubkowska, L., Kashlev, M., and Bustamante, C. (2009). Nucleosomal fluctuations govern the transcription dynamics of RNA polymerase II. Science 325, 626-628.
Ishibashi, T., Dryhurst, D., Rose, K.L., Shabanowitz, J., Hunt, D.F., and Ausio, J. (2009). Acetylation of vertebrate H2A.Z and its effect on the structure of the nucleosome. Biochemistry 48, 5007-5017.
Kaplan, N., Moore, I.K., Fondufe-Mittendorf, Y., Gossett, A.J., Tillo, D., Field, Y., LeProust, E.M., Hughes, T.R., Lieb, J.D., Widom, J., et al. (2009). The DNA-encoded nucleosome organization of a eukaryotic genome. Nature 458, 362-366.
Kelly, A.E., and Goulden, M.L. (2008). Rapid shifts in plant distribution with recent climate change. Proc Natl Acad Sci U S A 105, 11823-11826.
Kim, H.J., Hyun, Y., Park, J.Y., Park, M.J., Park, M.K., Kim, M.D., Lee, M.H., Moon, J., Lee, I., and Kim, J. (2004). A genetic link between cold responses and flowering time through FVE in Arabidopsis thaliana. Nat Genet 36, 167-171.
Kluth, A., Sprunck, S., Becker, D., Lorz, H., and Lutticke, S. (2002). 5' deletion of a gbss1 promoter region from wheat leads to changes in tissue and developmental specificities. Plant Mol Biol 49, 669-682.
Knezetic, J.A., and Luse, D.S. (1986). The presence of nucleosomes on a DNA template prevents initiation by RNA polymerase II in vitro. Cell 45, 95-104.
Kobor, M.S., Venkatasubrahmanyam, S., Meneghini, M.D., Gin, J.W., Jennings, J.L., Link, A.J., Madhani, H.D., and Rine, J. (2004). A protein complex containing the conserved Swi2/Snf2-related ATPase Swr1p deposits histone variant H2A.Z into euchromatin. PLoS Biol 2, E131.
Koini, M.A., Alvey, L., Allen, T., Tilley, C.A., Harberd, N.P., Whitelam, G.C., and Franklin, K.A. (2009). High temperature-mediated adaptations in plant architecture require the bHLH transcription factor PIF4. Curr Biol 19, 408-413.
Kovalchuk, N., Smith, J., Pallotta, M., Singh, R., Ismagul, A., Eliby, S., Bazanova, N., Milligan, A.S., Hrmova, M., Langridge, P., et al. (2009). Characterization of the wheat endosperm transfer cell-specific protein TaPR60. Plant Mol Biol 71, 81-98.
Krogan, N.J., Keogh, M.C., Datta, N., Sawa, C., Ryan, O.W., Ding, H., Haw, R.A., Pootoolal, J., Tong, A., Canadien, V., et al. (2003). A Snf2 family ATPase complex required for recruitment of the histone H2A variant Htz1. Mol Cell 12, 1565-1576.
Lam, F.H., Steger, D.J., and O'Shea, E.K. (2008). Chromatin decouples promoter threshold from dynamic range. Nature 453, 246-250.
Larkindale, J., and Vierling, E. (2008). Core genome responses involved in acclimation to high temperature. Plant Physiol 146, 748-761.
Lee, J.H., Yoo, S.J., Park, S.H., Hwang, I., Lee, J.S., and Ahn, J.H. (2007). Role of SVP in the control of flowering time by ambient temperature in Arabidopsis. Genes Dev 21, 397-402.
Lenoir, J., Gegout, J.C., Marquet, P.A., de Ruffray, P., and Brisse, H. (2008). A significant upward shift in plant species optimum elevation during the 20th century. Science 320, 1768-1771.
Li, B., Pattenden, S.G., Lee, D., Gutierrez, J., Chen, J., Seidel, C., Gerton, J., and Workman, J.L. (2005). Preferential occupancy of histone variant H2AZ at inactive promoters influences local histone modifications and chromatin remodeling. Proc Natl Acad Sci U S A 102, 18385-18390.
Li, M., Singh, R., Bazanova, N., Milligan, A.S., Shirley, N., Langridge, P., and Lopato, S. (2008). Spatial and temporal expression of endosperm transfer cell-specific promoters in transgenic rice and barley. Plant Biotechnol J 6, 465-476.
Lorch, Y., LaPointe, J.W., and Kornberg, R.D. (1987). Nucleosomes inhibit the initiation of transcription but allow chain elongation with the displacement of histones. Cell 49, 203-210.
Martin-Trillo, M., Lazaro, A., Poethig, R.S., Gomez-Mena, C., Pineiro, M.A., Martinez-Zapater, J.M., and Jarillo, J.A. (2006). EARLY IN SHORT DAYS 1 (ESD1) encodes ACTIN-RELATED PROTEIN 6 (AtARP6), a putative component of chromatin remodelling complexes that positively regulates FLC accumulation in Arabidopsis. Development 133, 1241-1252.
Mavrich, T.N., Jiang, C., Ioshikhes, I.P., Li, X., Venters, B.J., Zanton, S.J., Tomsho, L.P., Qi, J., Glaser, R.L., Schuster, S.C., et al. (2008). Nucleosome organization in the Drosophila genome. Nature 453, 358-362.
Meneghini, M.D., Wu, M., and Madhani, H.D. (2003). Conserved histone variant H2A.Z protects euchromatin from the ectopic spread of silent heterochromatin. Cell 112, 725-736.
Michael, T.P., Mockler, T.C., Breton, G., McEntee, C., Byer, A., Trout, J.D., Hazen, S.P., Shen, R., Priest, H.D., Sullivan, C.M., et al. (2008). Network discovery pipeline elucidates conserved time-of-day-specific cis-regulatory modules. PLoS Genet 4, e14.
Mittler, R. (2006). Abiotic stress, the field environment and stress combination. Trends Plant Sci 11, 15-19.
Mizuguchi, G., Shen, X., Landry, J., Wu, W.H., Sen, S., and Wu, C. (2004). ATP-driven exchange of histone H2AZ variant catalyzed by SWR1 chromatin remodeling complex. Science 303, 343-348.
Nguyen, V.Q., and Sil, A. (2008). Temperature-induced switch to the pathogenic yeast form of Histoplasma capsulatum requires Ryp1, a conserved transcriptional regulator. Proc Natl Acad Sci U S A 105, 4880-4885.
Petesch, S.J., and Lis, J.T. (2008). Rapid, transcription-independent loss of nucleosomes over a large chromatin domain at Hsp70 loci. Cell 134, 74-84.
Poethig, R.S. (2003). Phase change and the regulation of developmental timing in plants. Science 301, 334-336.
Polach, K.J., and Widom, J. (1995). Mechanism of protein access to specific DNA sequences in chromatin: a dynamic equilibrium model for gene regulation. J Mol Biol 254, 130-149.
Raisner, R.M., Hartley, P.D., Meneghini, M.D., Bao, M.Z., Liu, C.L., Schreiber, S.L., Rando, O.J., and Madhani, H.D. (2005). Histone variant H2A.Z marks the 5' ends of both active and inactive genes in euchromatin. Cell 123, 233-248.
Salome, P.A., and McClung, C.R. (2005). PSEUDO-RESPONSE REGULATOR 7 and 9 are partially redundant genes essential for the temperature responsiveness of the Arabidopsis circadian clock. Plant Cell 17, 791-803.
Samach, A., and Wigge, P.A. (2005). Ambient temperature perception in plants. Curr Opin Plant Biol 8, 483-486.
Segal, E., and Widom, J. (2009). From DNA sequence to transcriptional behaviour: a quantitative approach. Nat Rev Genet 10, 443-456.
Sung, D.Y., Vierling, E., and Guy, C.L. (2001). Comprehensive expression profile analysis of the Arabidopsis Hsp70 gene family. Plant Physiol 126, 789-800.
Sung, S., and Amasino, R.M. (2005). Remembering winter: toward a molecular understanding of vernalization. Annu Rev Plant Biol 56, 491-508.
Thambirajah, A.A., Dryhurst, D., Ishibashi, T., Li, A., Maffey, A.H., and Ausio, J. (2006). H2A.Z stabilizes chromatin in a way that is dependent on core histone acetylation. J Biol Chem 281, 20036-20044.
Till, B.J., Zerr, T., Comai, L., and Henikoff, S. (2006). A protocol for TILLING and Ecotilling in plants and animals. Nat Protoc 1, 2465-2477.
Tsankov, A.M., Brown, C.R., Yu, M.C., Win, M.Z., Silver, P.A., and Casolari, J.M. (2006). Communication between levels of transcriptional control improves robustness and adaptivity. Molecular systems biology 2, 65.
Whittle, C.M., McClinic, K.N., Ercan, S., Zhang, X., Green, R.D., Kelly, W.G., and Lieb, J.D. (2008). The genomic distribution and function of histone variant HTZ-1 during C. elegans embryogenesis. PLoS Genet 4, e1000187.
Wilczek, A.M., Roe, J.L., Knapp, M.C., Cooper, M.D., Lopez-Gallego, C., Martin, L.J., Muir, C.D., Sim, S., Walker, A., Anderson, J., et al. (2009). Effects of Genetic Perturbation on Seasonal Life History Plasticity. Science 323, 930-934.
Willis, C.G., Ruhfel, B., Primack, R.B., Miller-Rushing, A.J., and Davis, C.C. (2008). Phylogenetic patterns of species loss in Thoreau's woods are driven by climate change. Proc Natl Acad Sci U S A 105, 17029-17033.
Zhang, H., Roberts, D.N., and Cairns, B.R. (2005). Genome-wide dynamics of Htz1, a histone H2A variant that poises repressed/basal promoters for activation through histone loss. Cell 123, 219-231.
Zilberman, D., Coleman-Derr, D., Ballinger, T., and Henikoff, S. (2008). Histone H2A.Z and DNA methylation are mutually antagonistic chromatin marks. Nature 456, 125-129.

## Claims

1. A method for altering temperature sensing in a plant or a part thereof comprising modifying the presence or state of H2A.Z in the nucleosome by decreasing or increasing the level of H2A.Z in said plant or a part thereof or altering post-translational modification of H2A.Z, wherein said post-translational modification is acetylation.

2. The method according to claim 1 wherein said plant is selected from the group consisting of moncots and dicots, more preferably wherein said plant is a crop plant and most preferably wherein said crop plant is selected from the group consisting of rice, wheat, barley, soya, brassica, corn, rye, sorghum or sugarcane.

3. The method according to claim 1 wherein the presence of H2A.Z in the nucleosome is modified by decreasing the level of H2A.Z in said plant or a part thereof, said method comprising decreasing or inhibiting the expression of one or more nucleic acid sequence encoding H2A.Z, preferably wherein said nucleic acid is silenced using RNA interference.

4. The method according to claim 1 wherein the presence of H2A.Z in the nucleosome is modified by altering post-translational modification of H2A.Z said method comprising:
a) producing a mutant H2A.Z protein wherein said mutant H2A.Z protein has no acetylation sites, a reduced number of acetylation sites or increased acetylation sites wherein said acetylation site comprises a codon encoding a lysine (K) residue; or
b) altering the expression of a nucleic acid sequence encoding a histone deacetylase (HDAC) enzyme or altering the activity of HDAC; or
c) altering the expression of a nucleic acid sequence encoding a histone acetyltransferase (HAT)enzyme or altering the activity of HAT;
or combinations thereof.

5. The method according to claim 4 comprising producing a mutant H2A.Z protein said method comprising expressing a nucleic acid sequence encoding a H2A.Z protein wherein said nucleic acid sequence has been altered to
a. remove one or more acetylation sites, wherein said method comprises altering a codon encoding a lysine (K) residue in said nucleic acid sequence by replacing a nucleotide within said codon to produce a protein with non acetylatable residue; or
b. introduce one or more acetylation sites, wherein said method comprises altering a codon in said nucleic acid sequence by replacing or altering a nucleotide within said codon to produce a codon encoding a lysine (K)
wherein preferably, expression of said nucleic acid sequence is driven by the endogenous, a constitutive or tissue specific promoter.

6. The method according to claim 4 comprising
a. inhibiting or decreasing the expression of one or more genes encoding a HDAC enzyme to reduce or inhibit deacetylation of H2A.Z; or
b. expressing one or more nucleic acid sequences encoding a HDAC enzyme to increase removal of acetyl groups in H2A.Z, wherein preferably expression of said nucleic acid sequence is driven by the endogenous, a constitutive or tissue specific promoter.

7. The method according to claim 4 comprising
a. inhibiting or decreasing the expression of one or more genes encoding for a HAT enzyme to inhibit acetylation; or
b. expressing one or more nucleic acid sequences encoding a HAT enzyme to increase acetylation of H2A.Z, wherein preferably expression of said nucleic acid sequence is driven by the endogenous, a constitutive or tissue specific promoter.

8. A method for altering thermosensory responses in a plant said method comprising:
a) producing a mutant H2A.Z protein wherein said mutant H2A.Z protein has no acetylation sites, a reduced number of acetylation sites or increased acetylation sites, wherein said acetylation site comprises a codon encoding a lysine (K) residue; or
b) altering the expression of a nucleic acid sequence encoding a histone deacetylase (HDAC) enzyme or altering the activity of HDAC
or combinations thereof.

9. The method according to claim 8 wherein said thermosensory responses include up or downregulation of temperature dependent genes; preferably wherein said organism is a plant and said thermosensory responses include developmental responses, wherein said developmental response is one or more of the following responses: seed dormancy release, germination, hypocotyl elongation, petiole growth flowering, vernalisation, juvenile to adult transition, senescence, and temperature-protective responses.

10. A method for inhibiting the response of a plant to an increase in ambient temperature comprising increasing the presence of H2A.Z in the nucleosome or altering post-translational modification of H2A.Z wherein said post-translational modification is acetylation.

11. A plant H2A.Z molecule in which some or all of the lysines of said molecule have been replaced with non-acetylatable amino acid residues.

12. A plant comprising the H2A.Z according to claim 11 or a nucleic acid sequence encoding said H2A.Z.

13. A plant comprising:
a) a non-acetylatable version of histone H2A.Z;
b) an over-expressed histone deacetylase enzyme sufficient to remove histone acetylation marks in H2A.Z; or combinations thereof.

14. The plant H2A.Z of claim 11 or the plant of claim 12 or 13 wherein said plant is selected from the group consisting of monocots and dicots.

15. The plant H2A.Z or the plant of claim 14 wherein said plant is a crop plant.

16. The plant H2A.Z or the plant of claim 15 wherein said crop plant is selected from the group consisting of rice, wheat, barley, soya, brassica, corn, rye, sorghum or sugarcane.

## Patentansprüche

1. Verfahren zur Veränderung der Temperaturwahrnehmung in einer Pflanze oder einem Teil davon, umfassend Modifizieren des Vorhandenseins oder Zustands von H2A.Z im Nukleosom durch Verringern oder Erhöhen des Gehalts an H2A.Z in der Pflanze oder einem Teil davon oder Verändern posttranslationaler Modifikation von H2A.Z, wobei die posttranslationale Modifikation Acetylierung ist.

2. Verfahren nach Anspruch 1, wobei die Pflanze aus der Gruppe ausgewählt ist, die aus Einkeimblättrigen und Zweikeimblättrigen besteht, wobei besonders bevorzugt die Pflanze eine Nutzpflanze ist und wobei ganz besonders bevorzugt die Nutzpflanze aus der Gruppe ausgewählt ist, die aus Reis, Weizen, Gerste, Soja, Kohl, Mais, Roggen, Sorghum oder Zuckerrohr besteht.

3. Verfahren nach Anspruch 1, wobei das Vorhandensein von H2A.Z im Nukleosom modifiziert wird durch Verringern des Gehalts an H2A.Z in der Pflanze oder einem Teil davon, wobei das Verfahren Verringern oder Hemmen der Expression einer oder mehrerer für H2A.Z codierender Nukleinsäuresequenzen umfasst, wobei, vorzugsweise, die Nukleinsäure mittels RNA-Interferenz stillgelegt wird.

4. Verfahren nach Anspruch 1, wobei das Vorhandensein von H2A.Z im Nukleosom modifiziert wird durch Verändern posttranslationaler Modifikation von H2A.Z, wobei das Verfahren umfasst:
a) Produzieren eines mutanten H2A.Z-Proteins, wobei das mutante H2A.Z-Protein keine Acetylierungsstellen, eine reduzierte Anzahl von Acetylierungsstellen oder vermehrte Acetylierungsstellen aufweist, wobei die Acetylierungsstelle ein für einen Lysin- (K-) Rest codierendes Codon umfasst; oder
b) Verändern der Expression einer für ein Histon-Deacetylase- (HDAC-) Enzym codierenden Nukleinsäuresequenz oder Verändern der Aktivität von HDAC; oder
c) Verändern der Expression einer für ein Histon-Acetyltransferase- (HAT-) Enzym codierenden Nukleinsäuresequenz oder Verändern der Aktivität von HAT;
oder Kombinationen davon.

5. Verfahren nach Anspruch 4, umfassend Produzieren eines mutanten H2A.Z-Proteins, wobei das Verfahren Exprimieren einer für ein H2A.Z-Protein codierenden Nukleinsäuresequenz umfasst, wobei die Nukleinsäuresequenz verändert worden ist, um
a. eine oder mehrere Acetylierungsstellen zu entfernen, wobei das Verfahren Verändern eines für einen Lysin- (K-) Rest codierenden Codons in der Nukleinsäuresequenz durch Ersetzen eines Nukleotids innerhalb des Codons zwecks Produzierens eines Proteins mit nicht acetylierbarem Rest umfasst; oder
b. eine oder mehrere Acetylierungsstellen einzubringen, wobei das Verfahren Verändern eines Codons in der Nukleinsäuresequenz durch Ersetzen oder Verändern eines Nukleotids innerhalb des Codons zwecks Produzierens eines für ein Lysin (K) codierenden Codons umfasst,
wobei vorzugsweise die Expression der Nukleinsäuresequenz durch den endogenen, einen konstitutiven oder gewebespezifischen Promoter gesteuert wird.

6. Verfahren nach Anspruch 4, umfassend
a. Hemmen oder Verringern der Expression eines oder mehrerer für ein HDAC-Enzym codierender Gene, um Deacetylierung von H2A.Z zu reduzieren oder zu hemmen; oder
b. Exprimieren einer oder mehrerer für ein HDAC-Enzym codierender Nukleinsäuresequenzen, um Entfernung von Acetylgruppen in H2A.Z zu steigern, wobei vorzugsweise die Expression der Nukleinsäuresequenz durch den endogenen, einen konstitutiven oder gewebespezifischen Promoter gesteuert wird.

7. Verfahren nach Anspruch 4, umfassend
a. Hemmen oder Verringern der Expression eines oder mehrerer für ein HAT-Enzym codierender Gene, um Acetylierung zu hemmen; oder
b. Exprimieren einer oder mehrerer für ein HAT-Enzym codierender Nukleinsäuresequenzen, um Acetylierung von H2A.Z zu steigern, wobei vorzugsweise die Expression der Nukleinsäuresequenz durch den endogenen, einen konstitutiven oder gewebespezifischen Promoter gesteuert wird.

8. Verfahren zur Veränderung thermosensorischer Reaktionen in einer Pflanze, wobei das Verfahren umfasst:
a) Produzieren eines mutanten H2A.Z-Proteins, wobei das mutante H2A.Z-Protein keine Acetylierungsstellen, eine reduzierte Anzahl von Acetylierungsstellen oder vermehrte Acetylierungsstellen aufweist, wobei die Acetylierungsstelle ein für einen Lysin- (K-) Rest codierendes Codon umfasst; oder
b) Verändern der Expression einer für ein Histon-Deacetylase- (HDAC-) Enzym codierenden Nukleinsäuresequenz oder Verändern der Aktivität von HDAC,
oder Kombinationen davon.

9. Verfahren nach Anspruch 8, wobei die thermosensorischen Reaktionen Hoch- oder Herunterregulierung temperaturabhängiger Gene einschließen; wobei, vorzugsweise, der Organismus eine Pflanze ist und die thermosensorischen Reaktionen Entwicklungsreaktionen einschließen, wobei die Entwicklungsreaktion in einer oder mehreren der folgenden Reaktionen besteht: Samendormanzbrechung, Keimung, Hypokotyllängenwachstum, Petioluswachstum, Blühen, Vernalisation, Übergang vom Juvenil- zum Adultstadium, Seneszenz und temperaturprotektiven Reaktionen.

10. Verfahren zur Hemmung der Reaktion einer Pflanze auf eine Zunahme der Umgebungstemperatur, umfassend Vermehren des Vorhandenseins von H2A.Z im Nukleosom oder Verändern posttranslationaler Modifikation von H2A.Z, wobei die posttranslationale Modifikation Acetylierung ist.

11. Pflanzen-H2A.Z-Molekül, in dem einige oder alle der Lysine des Moleküls durch nicht acetylierbare Aminosäurereste ersetzt worden sind.

12. Pflanze, umfassend das H2A.Z nach Anspruch 11 oder eine für das H2A.Z codierende Nukleinsäuresequenz.

13. Pflanze, umfassend:
a) eine nicht acetylierbare Version von Histon H2A.Z;
b) ein überexprimiertes Histon-Deacetylase-Enzym, das ausreicht, um Histon-Acetylierungs-Markierungen in H2A.Z zu entfernen;
oder Kombinationen davon.

14. Pflanzen-H2A.Z nach Anspruch 11 oder Pflanze nach Anspruch 12 oder 13, wobei die Pflanze aus der Gruppe ausgewählt ist, die aus Einkeimblättrigen und Zweikeimblättrigen besteht.

15. Pflanzen-H2A.Z oder Pflanze nach Anspruch 14, wobei die Pflanze eine Nutzpflanze ist.

16. Pflanzen-H2A.Z oder Pflanze nach Anspruch 15, wobei die Nutzpflanze aus der Gruppe ausgewählt ist, die aus Reis, Weizen, Gerste, Soja, Kohl, Mais, Roggen, Sorghum oder Zuckerrohr besteht.

## Revendications

1. Procédé destiné à modifier la perception de température dans une plante ou partie de celle-ci, comprenant modifier la présence ou l'état de H2A.Z dans le nucléosome en diminuant ou en augmentant le niveau de H2A.Z dans ladite plante ou une partie de celle-ci ou en modifiant la modification post-traduction de H2A.Z, où ladite modification post-traduction est l'acétylation.

2. Procédé selon la revendication 1, dans lequel ladite plante est sélectionnée parmi le groupe consistant en monocotylédones et en dicotylédones, plus préférentiellement ladite plante est une plante de culture et le plus préférentiellement où ladite plante de culture est sélectionnée parmi le groupe consistant en riz, blé, orge, soja, chou, maïs, seigle, sorgho ou canne à sucre.

3. Procédé selon la revendication 1, dans lequel la présence de H2A.Z dans le nucléosome est modifiée en diminuant le niveau de H2A.Z dans ladite plante ou une partie de celle-ci, ledit procédé comprenant diminuer ou inhiber l'expression d'une ou de plusieurs séquences d'acide nucléique codant H2A.Z, de préférence où ledit acide nucléique est éteint en utilisant l'interférence par ARN.

4. Procédé selon la revendication 1, dans lequel la présence de H2A.Z dans le nucléosome est modifiée en modifiant la modification post-traduction de H2A.Z, ledit procédé comprenant:
a) produire une protéine H2A.Z mutante où ladite protéine H2A.Z mutante n'a pas de site d'acétylation, un nombre réduit de sites d'acétylation ou des sites d'acétylation augmentés, où ledit site d'acétylation comprend un codon codant un résidu lysine (K); ou bien
b) modifier l'expression d'une séquence d'acide nucléique codant une enzyme histone désacétylase (HDAC) ou modifier l'activité de HDAC; ou bien
c) modifier l'expression d'une séquence d'acide nucléique codant une enzyme histone acétyltransférase (HAT) ou modifier l'activité de HAT;
ou des combinaisons de ceci.

5. Procédé selon la revendication 4, comprenant produire une protéine H2A.Z mutante, ledit procédé comprenant exprimer une séquence d'acide nucléique codant une protéine H2A.Z, où ladite séquence d'acide nucléique a été modifiée pour
a. retirer un ou plusieurs sites d'acétylation, où ledit procédé comprend modifier un codon codant un résidu lysine (K) dans ladite séquence d'acide nucléique en remplaçant un nucléotide dans ledit codon pour produire une protéine avec un résidu non acétylable; ou bien
b. introduire un ou plusieurs sites d'acétylation, où ledit procédé comprend modifier un codon dans ladite séquence d'acide nucléique en remplaçant ou en modifiant un nucléotide dans ledit codon pour produire un codon codant une lysine (K)
dans lequel de préférence, l'expression de ladite séquence d'acide nucléique est entraînée par le promoteur endogène, un promoteur constitutif ou spécifique de tissu.

6. Procédé selon la revendication 4, comprenant:
a. inhiber ou diminuer l'expression d'un ou de plusieurs gènes codant une enzyme HDAC afin de réduire ou d'inhiber la désacétylation de H2A.Z; ou bien
b. exprimer une ou plusieurs séquences d'acide nucléique codant une enzyme HDAC afin d'augmenter l'enlèvement de groupes acétyle dans H2A.Z, où de préférence l'expression de ladite séquence d'acide nucléique est entraînée par le promoteur endogène, un promoteur constitutif ou spécifique de tissu.

7. Procédé selon la revendication 4, comprenant:
a. inhiber ou diminuer l'expression d'un ou de plusieurs gènes codant pour une enzyme HAT afin d'inhiber l'acétylation; ou bien
b. exprimer une ou plusieurs séquences d'acide nucléique codant une enzyme HAT afin d'augmenter l'acétylation de H2A.Z, où de préférence l'expression de ladite séquence d'acide nucléique est entraînée par le promoteur endogène, un promoteur constitutif ou spécifique de tissu.

8. Procédé destiné à modifier les réponses thermosensorielles dans une plante, ledit procédé comprenant:
a) produire une protéine H2A.Z mutante où ladite protéine H2A.Z mutante n'a pas de site d'acétylation, un nombre réduit de sites d'acétylation ou des sites d'acétylation augmentés, où ledit site d'acétylation comprend un codon codant un résidu lysine (K); ou bien
b) modifier l'expression d'une séquence d'acide nucléique codant une enzyme histone désacétylase (HDAC) ou modifier l'activité de HDAC
ou des combinaisons de ceci.

9. Procédé selon la revendication 8, dans lequel lesdites réponses thermosensorielles comprennent la régulation à la hausse ou à la baisse de gènes dépendants de la température; de préférence où ledit organisme est une plante et lesdites réponses thermosensorielles comprennent des réponses de développement, où ladite réponse de développement est l'une ou plusieurs des réponses suivantes: déblocage de la dormance des graines, germination, allongement de l'hypocotyle, croissance des pétioles, épanouissement, vernalisation, transition de juvénile en adulte, sénescence et réponses de protection contre la température.

10. Procédé destiné à inhiber la réponse d'une plante à une hausse de température ambiante comprenant augmenter la présence de H2A.Z dans le nucléosome ou modifier la modification post-traduction de H2A.Z, où ladite modification post-traduction est l'acétylation.

11. Molécule H2A.Z végétale dans laquelle certaines ou toutes les lysines de ladite molécule ont été remplacées par des résidus d'acides aminés non acétylables.

12. Plante comprenant la H2A.Z selon la revendication 11 ou une séquence d'acide nucléique codant ladite H2A.Z.

13. Plante comprenant:
a) une version non acétylable d'histone H2A.Z;
b) une enzyme histone désacétylase surexprimée suffisante pour retirer les marques d'acétylation d'histone dans H2A.Z;
ou des combinaisons de celles-ci.

14. H2A.Z végétale selon la revendication 11 ou plante selon la revendication 12 ou 13, où ladite plante est sélectionnée parmi le groupe consistant en monocotylédones et dicotylédones.

15. H2A.Z végétale ou plante selon la revendication 14, où ladite plante est une plante de culture.

16. H2A.Z végétale ou plante selon la revendication 15, où ladite plante de culture est sélectionnée parmi le groupe consistant en riz, blé, orge, soja, chou, maïs, seigle, sorgho ou canne à sucre.
